(19) Europäisches Patentamt / European Patent Office / Office européen des brevets

(11) **EP 3 062 755 B1**

(12) **EUROPEAN PATENT SPECIFICATION**

(45) Date of publication and mention
of the grant of the patent:
**05.02.2020 Bulletin 2020/06**

(51) Int Cl.:
*A61F 13/62* (2006.01)          *A44B 18/00* (2006.01)
*A61F 13/56* (2006.01)          *A61F 13/58* (2006.01)

(21) Application number: **14799615.1**

(86) International application number:
**PCT/US2014/062958**

(22) Date of filing: **29.10.2014**

(87) International publication number:
**WO 2015/066210 (07.05.2015 Gazette 2015/18)**

(54) **FASTENING LAMINATE AND METHOD OF MAKING THE SAME**

BEFESTIGUNGSLAMINAT UND VERFAHREN ZUR HERSTELLUNG DAVON

STRATIFIÉ AVEC FIXATION ET SON PROCÉDÉ DE FABRICATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.10.2013 US 201361897588 P**

(43) Date of publication of application:
**07.09.2016 Bulletin 2016/36**

(73) Proprietor: **3M Innovative Properties Company
St. Paul, MN 55133-3427 (US)**

(72) Inventors:
• **GILBERT, Thomas J.**
  **Saint Paul, Minnesota 55133-3427 (US)**
• **PELTIER, Mark A.**
  **Saint Paul, Minnesota 55133-3427 (US)**
• **LUEPKE, Ryan M.**
  **Saint Paul, Minnesota 55133-3427 (US)**
• **NELSON, Todd L.**
  **Saint Paul, Minnesota 55133-3427 (US)**
• **EYNON, Stuart L.**
  **Bracknell**
  **Berkshire RG12 8HT (GB)**

(74) Representative: **Vossius & Partner
Patentanwälte Rechtsanwälte mbB
Siebertstrasse 3
81675 München (DE)**

(56) References cited:
**WO-A1-2012/112768      US-A1- 2007 173 781
US-A1- 2012 330 266**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**BACKGROUND**

Mechanical fasteners, which are also called hook and loop fasteners, are useful for providing releasable attachment in numerous applications. For example, mechanical fasteners are widely used in wearable absorbent articles to fasten such articles around the body of a person. In typical configurations, a hook strip or patch on a fastening tab attached to the rear waist portion of a diaper or incontinence garment, for example, can fasten to a landing zone of loop material on the front waist region, or the hook strip or patch can fasten to the backsheet (e.g., nonwoven backsheet) of the diaper or incontinence garment in the front waist region. Absorbent articles often employ woven or nonwoven materials, for example, to provide a cloth-like feeling in order to increase the comfort of wearing.

Fastening tabs often have a manufacturer's end that is attached to the rear waist region of an absorbent article and a user's end that can be grasped and extends outwardly beyond the edge of an absorbent article before it is attached to the front waist region of the absorbent article. The attachment point of the tab at the manufacturer's end must be strong enough to withstand the force applied during the application and wearing of the absorbent article; otherwise the tab can separate from the absorbent article during use. So called Y-bonded fastening tabs are proposed to have strong attachment to absorbent articles; see, e.g., U.S. Pat. No. 3,848,594 (Buell).

In some cases, fastening tabs include a substrate that is coated entirely with adhesive. The adhesive at the manufacturer's end is used to permanently attach the fastening tab to the edge of the absorbent article, and the adhesive at the user's end is used to attach the hook strip or patch to the fastening tab. Exposed adhesive between the edge of the absorbent article and the hook strip or patch can be managed by means of a release tape, for example, on a surface of the absorbent article that comes into contact with the exposed adhesive while the absorbent article is in the package.

Some hook members have been made with openings in the backing from which the hooks project. See, e.g., U. S. Pat. Nos. 4,001,366 (Brumlik) and 7,407,496 (Peterson), U.S. Pat. Appl. Pub. No. 2012/0204383 (Wood et al.), and Int. Pat. Appl. Pub. Nos. WO 2005/122818 (Ausen et al.) and WO 1994/02091 (Hamilton). Also, laminates with separated mechanical fastening strips are described in U.S. Pat. Appl. Pub. No. 2007/0039142 (Petersen et al.) and Int. Pat. Appl. Pub. No. WO2011/163020 (Hauschildt et al.).

Some nonwoven materials have been made with openings. Such nonwovens have been attached to elastics or extensible pleated backings. See, e.g., U. S. Pat. Appl. Pub. No. 2004/0147890 (Nakahata et al.), Int. Pat. Appl. Pub. No. WO 1996/10481 (Abuto et al.), and European Patent No. EP 1066008 B1 (Eaton et al.). A reticulated mechanical fastening patch having loops is described in U.S. Pat. Appl. Pub. No. 2012/0330266 (Zonneveld et al.).

WO 2012/112768 A1 discloses a method of making a mechanical fastener and a reticulated mechanical fastener.

US 2007/0173781 A1 discloses a closure tape tab for an absorbent article, particularly for a disposable diaper, for fastening the article on the body of a person. The closure tape tab comprises a proximal end portion and a distal end portion being connected by an inner tab portion, wherein the inner tab portion has a first major surface and a second major surface. The proximal and distal end portions are connected to the inner tab portion and the first major surface thereof such that opposing ends of the proximal and distal end portions are spaced apart from each other. The distal end portion is folded over toward the proximal end portion such that at least a part of the first major surface of the inner tab portion in the space is covered.

**SUMMARY**

A fastening laminate and a method as recited in the independent claims are provided. The dependent claims define embodiments.

The present disclosure provides a fastening laminate that includes a substrate bearing a combination of adhesive and mechanical fasteners. The fastening laminate includes a release tape that has one end attached to the substrate. The release tape is folded so that its opposite end covers some, but not all, of the adhesive and mechanical fasteners with a release surface. The portion of the fastening laminate that is covered by the release tape has more adhesive fastening surface available than the portion of the fastening laminate not covered by the release tape.

When this fastening laminate is attached to the chassis of a diaper and folded over on a diaper manufacturing line, the substrate is typically attached the backsheet side of the diaper, and the release tape is typically attached to the topsheet side of the diaper. In this configuration, the adhesive surface covered by the release tape is protected from the typically fibrous surface of the diaper. However, at least a portion of the mechanical fastener(s) and optionally adhesive are not covered by release tape and can engage the fibers of the diaper, for example, to prevent the fastening laminate from popping open.

In one aspect, the present disclosure provides a fastening laminate including a substrate with an adhesive layer disposed thereon; a mechanical fastener on the adhesive layer; and a release tape having a release surface. The release tape has an end portion attached to the substrate closer to a first portion of the mechanical fastener than a second portion of the mechanical fastener. The mechanical fastener includes an opening that reveals a first portion of the adhesive layer. When the release tape is in contact with at least some of the adhesive layer, the release surface covers the first portion of the mechanical fastener, at least partially covers the first portion of the adhesive layer, but does not completely cover the second portion of the mechanical fastener. The first portion of the adhesive layer is between the first portion of the mechanical fastener and the second portion of the mechanical fastener.

In some embodiments of the foregoing aspects, the first mechanical fastening portion (or first portion of the mechanical fastener) is one of multiple first mechanical fastening portions provided by strands of the backing attached to each other at intact bridging regions in the backing and separated from each other between the bridging regions to form multiple first openings that reveal multiple first portions of the adhesive layer not covered by the first mechanical fastening portions, and the second mechanical fastening portion (or second portion of the mechanical fastener) is one of multiple second mechanical fastening portions provided by strands of the backing attached to each other at intact bridging regions in the backing and separated from each other between the bridging regions to form second openings that reveal multiple second portions of the adhesive layer not covered by the second mechanical fastening portions. The second openings are typically smaller than the first openings. The degree of separation of the strands may be adjusted based upon, for example, the desired appearance, desired amount of exposed adhesive, weight, or cost in the final product.

In another aspect, the present disclosure provides an absorbent article including the fastening laminate described above. The substrate and a second surface of the release tape, opposite the release surface, are attached along one edge of the absorbent article.

In another aspect, the present disclosure provides a method of making a fastening laminate. The method includes providing an adhesive layer on a substrate, attaching a mechanical fastener to the adhesive layer, and attaching a release tape having a release surface to the adhesive layer closer to a first portion of the mechanical fastener than a second portion of the mechanical fastener. The mechanical fastener includes a first opening that reveals a first portion of the adhesive layer. The release surface covers the first portion of the mechanical fastener, at least partially covers the first portion of the adhesive layer, but does not completely cover the second portion of the mechanical fastener. The first portion of the adhesive layer is between the first portion of the mechanical fastener and the second portion of the mechanical fastener.

For any of the above aspects, a first ratio of a surface area of the first mechanical fastening portion(s) to a surface area of the first portion(s) of the adhesive layer is smaller than a second ratio of a surface area of the second mechanical fastening portion(s) to a surface area of any exposed adhesive on an opposite side the second mechanical fastening portion from the first portion(s) of the adhesive layer.

The combination of adhesive and mechanical fasteners is useful, for example, for providing good shear and peel performance, for providing good closure for soiled absorbent articles for disposal, and for providing a cost benefit. However, adhesives can be damaging to the outer surface of an absorbent article. Release tape used to manage the adhesive attachment to the absorbent article can result in popping open of a fastening tab on a manufacturing line. The fastening laminate disclosed herein balance these advantages and disadvantages by covering at least some of exposed adhesive with release tape while leaving at least some mechanical fastener and optionally adhesive available to prevent the fastening laminate from popping open on a manufacturing line, for example.

In this application, terms such as "a", "an" and "the" are not intended to refer to only a singular entity, but include the general class of which a specific example may be used for illustration. The terms "a", "an", and "the" are used interchangeably with the term "at least one". The phrases "at least one of' and "comprises at least one of' followed by a list refers to any one of the items in the list and any combination of two or more items in the list. All numerical ranges are inclusive of their endpoints and non-integral values between the endpoints unless otherwise stated.

The terms "first" and "second" are used in this disclosure. It will be understood that, unless otherwise noted, those terms are used in their relative sense only. For these components, the designation of "first" and "second" may be applied to directions, features, or components merely as a matter of convenience in the description of one or more of the embodiments.

The terms "multiple" and "a plurality" refer to more than one.

The term "opening" should be understood to be a void space in the mechanical fastener that is surrounded by portions of the mechanical fastener. An opening provides a void in the mechanical fastener but typically does not separate the mechanical fastener into discrete portions. One opening can be enclosed by two of the multiple strands of the backing as described for some of the embodiments disclosed herein.

The above summary of the present disclosure is not intended to describe each disclosed embodiment or every implementation of the present disclosure. The description that follows more particularly exemplifies illustrative embodiments. It is to be understood, therefore, that the drawings and following description are for illustration purposes only and should not be read in a manner that would unduly limit the scope of this disclosure.

## BRIEF DESCRIPTION OF THE DRAWINGS

The disclosure may be more completely understood in consideration of the following detailed description of various embodiments of the disclosure in connection with the accompanying drawings, in which:

FIG. 1 is a side view of an embodiment of a fastening laminate according to the present disclosure installed on an absorbent article and configured for storage;

FIG. 2 is a side view of another embodiment of a fastening laminate according to the present disclosure before it is installed on an absorbent article;

FIG. 3A is a top view of an embodiment of a portion of a slit web useful for making mechanical fastening portions of the fastening laminate according to the present disclosure;

FIG. 3B is a top view of a portion of the slit web shown in FIG. 3A after it is spread non-uniformly;

FIG. 4A is a schematic top view of another embodiment of a portion of a slit web useful for making mechanical fastening portions of the fastening laminate according to the present disclosure;

FIG. 4B is a schematic top view of the portion of the slit web of FIG. 4A after it is spread to provide mechanical fastening portions;

FIG. 5A is a schematic top view of yet another embodiment of a portion of a slit web useful for making mechanical fastening portions of the fastening laminate according to the present disclosure;

FIG. 5B is a schematic top view of the portion of the slit web of FIG. 5A after it is spread to provide mechanical fastening portions;

FIG. 6 is a top view of yet another embodiment of a fastening laminate according to the present disclosure;

FIG. 7 is a side view of still another embodiment of a fastening laminate according to the present disclosure before it is installed on an absorbent article;

FIG. 8 is top view of the fastening laminate shown in FIG. 7; and

FIG. 9 is a side view of still another embodiment of a fastening laminate according to the present disclosure before it is installed on an absorbent article.

## DETAILED DESCRIPTION

Reference will now be made in detail to embodiments of the disclosure, one or more examples of which are illustrated in the drawings. Features illustrated or described as part of one embodiment can be used with other embodiments to yield still a third embodiment. It is intended that the present disclosure include these and other modifications and variations.

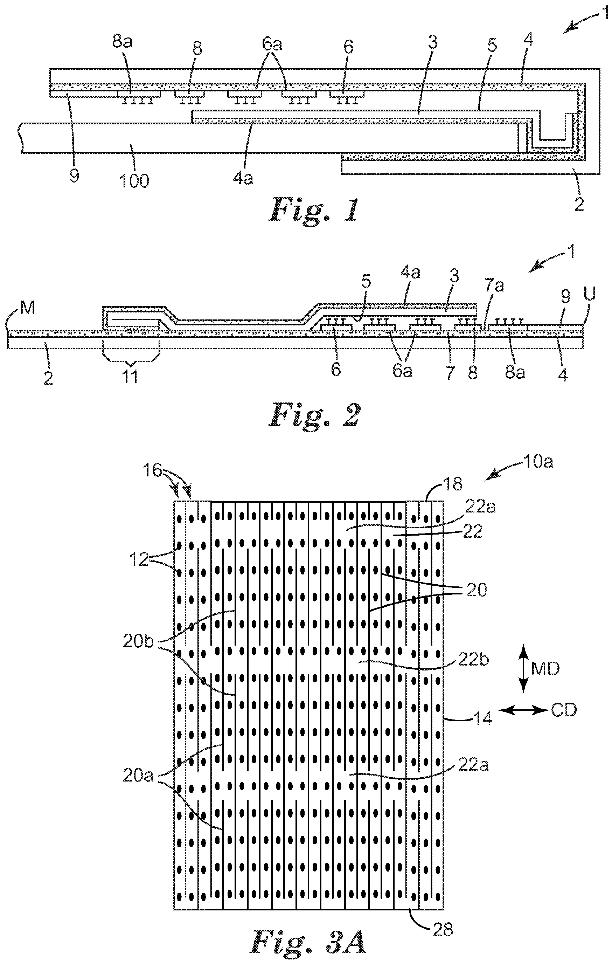

FIG. 1A illustrates an embodiment of a fastening laminate 1 according to the present disclosure installed on an absorbent article 100. The fastening laminate 1 is in a configuration that is common while an absorbent article 100 (e.g., a diaper) is being stored. In the illustrated storage configuration, one end the fastening laminate's substrate 2 is adhered to one side, typically the backsheet side, of the absorbent article 100 in a manner typically designed to provide permanent attachment through adhesive 4. The opposite end of the fastening laminate is wrapped around the edge of the absorbent article 100 to its opposite side, typically the top sheet side, where it is releasably attached. Fastening laminates according to the present disclosure have a combination of adhesive 4 and mechanical fasteners 6, 6a, 8, and 8a. The mechanical fasteners 8 and 8a can releasably attach to the top sheet side of an absorbent article. Release tape 3 is provided to allow the adhesive 4 to release from the top sheet side of the absorbent article. In the illustrated embodiment, the release tape 3 is adhered to the absorbent article 100 with adhesive 4a and has an adhesive-to-adhesive connection to the substrate 2. The configuration of the release tape 3 and substrate 2 forms a so-called Y-bond.

It has been reported in U.S. Pat. No. 4,067,337 (Ness) that a plastic mesh can be placed over an adhesive layer on a fastening tab to prevent the adhesive from adhering too strongly and ripping the outer cover of the diaper. It was proposed that such a plastic mesh could eliminate the need for a release sheet.

In view of the observations of Ness, it may be expected that a mechanical fastener adhered to an adhesive layer on a fastening tab, in which the mechanical fastener has openings that expose a portion of the adhesive layer, may not require a release tape for releasable fastening. However, we have discovered that depending on the size of the openings between mechanical fastening portions, a fastening tab in the storage configuration shown in FIG. 1 can be very difficult to open, for example, if the absorbent articles are compression packed for a few hours. To solve this problem, laminates according to the present disclosure include a release tape 3 to prevent the exposed adhesive in the openings of the mechanical fastener from adhering too strongly to the edge of the absorbent article. However, there are still exposed male fastening elements and, in some embodiments, some exposed adhesive that can provide an anti-pop-open feature for the fastening laminate disclosed herein.

FIG. 2 illustrates a fastening laminate similar to FIG. 1 but before it is installed on an absorbent article. FIG. 2 illustrates an embodiment of the fastening laminate as it may appear when it is initially manufactured. Manufacturing end "M" is the end that is permanently attached to the absorbent article 100, and user's end "U" can be releasably attached to the absorbent article 100 during storage or use. Referring now to FIGS. 1 and 2, fastening laminate 1 includes a substrate 2 provided with a layer of adhesive 4. First mechanical fastening portions 6, 6a and second mechanical fastening portions 8 and 8a are adhered to the substrate 2 using the adhesive layer 4. A first portion of the adhesive layer 7 between the first and second mechanical fastening portions 6, 6a, 8 is not covered by the first and second mechanical fastening portions 6, 6a, 8. Film 9, useful as a fingerlift, is also adhered to the substrate 2 using the adhesive layer 4. Closer to the manufacturing end "M" than the user's end "U" of the fastening laminate 1, an end portion 11 of the release tape 3 is attached to substrate 2. The end portion 11 is defined by a first edge and a fold line. The release tape 3 has a release surface 5, and, in the illustrated embodiment, it is coated with an adhesive 4a, which allows it to be attached to the absorbent article 100 as shown in FIG. 1. In FIG. 2, the release tape 3 is folded back on itself at the fold line, and the release surface 5 covers at least some of the first mechanical portions 6, 6a and the first portion of the adhesive layer 7 between the first and second mechanical fastening portion but does not cover the second mechanical fastening portion 8.

When the release surface 5 is said to cover the first mechanical fastening portion 6,6a and the first portion of the adhesive layer 7, in any of the embodiments disclosed herein, typically the first mechanical fastening portion 6,6a is completely covered by the release surface. In some embodiments, the first portion of the adhesive layer 7 is completely covered, but in some embodiments, the first portion of the adhesive layer 7 is at least partially covered. For example, there may be a portion near the edge of the second mechanical fastening portion 8 that is uncovered by the release tape. When the release surface 5 is said not to completely cover the second mechanical fastening portion 8, in any of the embodiments disclosed herein, typically the second mechanical fastening portion 8 remains at least partially uncovered. For example, as shown in FIGS. 1 and 2, the release surface may overlap one side of the second mechanical fastening portion 8, but still be said not to cover the second mechanical fastening portion 8. In this context, in any of the embodiments disclosed herein, it may be said that the release surface 5 does not completely cover the second mechanical fastening portion 8. In some embodiments, the second mechanical fastening portion (8a, for example) is completely uncovered by the release surface.

In some embodiments, including the embodiments illustrated herein, the first mechanical fastening portion is one of multiple first mechanical fastening portions. In some embodiments, including the embodiments illustrated in FIGS. 1, 2, and 6, the second mechanical fastening portion is one of multiple second mechanical fastening portions. Generally, when there are multiple "first" mechanical fastening portions covered by release tape, the "first" mechanical fastening portion may be construed as the one farthest away from the second mechanical fastening portion 8, 8a, 2008 or any of the first mechanical fastening portions 6, 6a, 2006, 2006a that has adhesive between itself and the second mechanical fastening portion 8, 8a, 2008. Likewise when there are multiple "second" mechanical fastening portions not completely covered by release tape, the "second" mechanical fastening portion may be construed as the second mechanical fastening portion 8a closest to one end of the fastening laminate, generally the user's end "U", or any of the second mechanical fastening portions 8, 8a, 2008 that has adhesive between itself and the first mechanical fastening portion 6,6a, 2006, 2006a.

In the embodiments illustrated in FIGS. 1 and 2, the fastening laminate 1 includes a second portion of the adhesive layer 7a not covered by the second mechanical fastening portions 8 and 8a, and the first and second portions of the adhesive layer 7, 7a, respectively, are on opposite sides of the second mechanical fastening portion 8. Since the second mechanical fastening portion 8 is not completely covered by the release surface 5, is should be understood that because the second portion of the adhesive layer is on a side of the second mechanical fastening portion opposite the first portion of the adhesive layer, the second portion of the adhesive layer 7a is completely uncovered by the release surface. The second portion of the adhesive layer 7a is therefore advantageously available to engage the fibers of the absorbent article 100, for example, to prevent the fastening laminate 1 from popping open on a manufacturing line for the absorbent article. However, typically the surface area of the second portion of the adhesive layer 7a is small enough such that it can be released from the absorbent article without requiring excessive force.

In any of the embodiments of the fastening laminate disclosed herein that includes a second portion of the adhesive layer, a first ratio of a surface area of the first mechanical fastening portion to a surface area of the first portion of the adhesive layer may be smaller than a second ratio of a surface area of the second mechanical fastening portion to a surface area of the second portion of the adhesive layer. In embodiments in which there are multiple first and second mechanical fastening portions as shown in FIGS. 1 and 2, the surface area of the first mechanical fastening portion refers to the combined surface area of all the fastening portions 6,6a, which may be considered the combined surface area of all of the first mechanical fastening portions completely covered by the release surface 5. Similarly, the surface area of the first portion of the adhesive layers refers to the combined surface area of all the first portions of the adhesive layers 7 that are at least partially covered by the release surface 5. Similarly, the surface area of the second mechanical fastening portion refers to the combined surface area of the all the second mechanical fastening portions 8, 8a that are at least partially uncovered by the release surface 5. And, the surface area of the second portion of the adhesive layer refers to the combined surface area of all the second portions of the adhesive layers 7a between the first mechanical fastening portion 6,6a and the user's end U that are completely uncovered by the release surface 5. In some embodiments, the first ratio of the surface area of the first mechanical fastening portion(s) to the surface area of first portion(s) of the adhesive layer is up to 2.5:1, 2:1, 1.3:1, 1:1, 1:2.5, 1:2, 1:3 or less. In some embodiments, the second ratio of the surface area of the second mechanical fastening portion(s) to the surface area of second portion(s) of the adhesive layer is up to 2:1, 3:1, 4:1, 5:1, 6.67:1, 10:1, 20:1, or more.

In some embodiments of the fastening laminate disclosed herein, none of the adhesive layer is exposed to the side of the second mechanical fastening portion opposite the first portion of the adhesive layer. That is, in the portion of the user's end U not covered by the release surface 5, the adhesive layer 4 may be completely covered by the second mechanical fastening portion 8 and optionally fingerlift 9. In these embodiments, the second mechanical fastening portion may provide enough engagement with the fibers of the absorbent article 100 to prevent the fastening laminate from popping open.

In some embodiments, the first mechanical fastening portion(s) 6, 6a and/or the second mechanical fastening portion(s) 8,8a are part of a mechanical fastener having an opening that reveals a portion of the adhesive layer not covered by the first mechanical fastening portion(s) or the second mechanical fastening portion, respectively. For these embodiments, FIGS. 1 and 2 illustrate examples of a cross section taken through multiple openings in the mechanical fastener. The openings may be formed by any suitable method, for example, die punching, extrusion forming, or slitting a backing having upstanding fastening elements and spreading or stretching the backing to form the openings. In these embodiments, the first and second mechanical fastening portions may be connected to each other and provided from a single piece of mechanical fastener. That is, the mechanical fastener may be an intact piece that includes first and second mechanical fastening portions, and the first and second mechanical fastening portions are not discrete portions. In some embodiments, the first and/or the second mechanical fastening portions include the backing with the openings formed by strands of the backing attached to each other at intact bridging regions in the backing and separated from each other between the bridging regions. The strands and openings in these embodiments may be formed, for example, by making interrupted slits in the backing and then spreading the backing to a desired degree.

Various embodiments of a slit web before and after spreading useful for making first and second mechanical fastening portions for the fastening laminates disclosed herein are illustrated in FIGS. 3A, 3B, 4A, 4B, 5A, and 5B and described below. In any of these embodiments, the term "web" can refer to a continuous or running web, sometimes having an indefinite length. A web can typically be handled in a roll-to-roll process. The term "machine direction" (MD) as used above and below denotes the direction of a running web of material during a manufacturing process. When a strip is cut from a continuous web, the machine direction corresponds to the length of the strip. As used herein, the terms "machine direction" and "longitudinal direction" are typically used interchangeably. The term "cross-machine direction" (CD) as used above and below denotes the direction which is essentially perpendicular to the machine direction. When a strip is cut from a continuous web, the cross-machine direction corresponds to the width "W" of the strip.

FIG. 3A illustrates an example of a portion of a slit web 10a with interrupted slits 20 that is spread in some embodiments of the fastening laminate disclosed herein. In the illustrated embodiment, the slit web 10a includes mechanical fastening elements, which are male fastening elements 12. Illustrated slit web 10a has a thermoplastic backing 14 with multiple rows 16 of male fastening elements 12 projecting from a first surface of the backing 14. The first surface of the backing is the surface that is visible in FIG. 3A. The first surface (that is, the surface with mechanical fastening elements) can also be called the first major surface in any of the embodiments disclosed herein. In the illustrated embodiment, the multiple rows 16 of male fastening elements 12 are aligned in the MD although this is not a requirement. The term "row" refers to male fastening elements lined up in a particular direction. The row or line of male fastening elements may be substantially straight.

In the portion of slit web 10a, interrupted slits 20 are cut into the backing between some pairs of adjacent rows 16 of male fastening elements 12 although this is not a requirement. When an interrupted slit is cut between adjacent rows of male fastening elements 12, it typically means that the particular slit does not cross over a row of male fastening elements 12. The illustrated interrupted slits 20 are linear in the same direction as the multiple rows 16, which in the illustrated embodiment is the MD, and extend from the top edge 18 to the bottom edge 28 of the backing 14. The interrupted slits are interrupted by intact bridging regions 22 of the backing 14. The bridging regions 22 are regions where the web is not cut through, and at least a portion of the bridging regions 22 can be considered collinear with interrupted slit 20. The intact bridging regions 22 divide the interrupted slits into a series of spaced apart slit portions 20a. The spaced apart slit portions 20a and 20b and consequently bridging regions 22a and 22b of adjacent interrupted slits are staggered in a direction "CD" perpendicular to the direction "MD" of the interrupted slits 20. The bridging regions are staggered such that bridging region 22b is located substantially midway between bridging regions 22a in the direction "MD". However, in some embodiments, the upstanding posts 12, interrupted slits 20, and bridging regions 22, 22a, and 22b may be positioned in other arrangements. When the slit portions and bridging regions are staggered, slit web 10a can be readily spread.

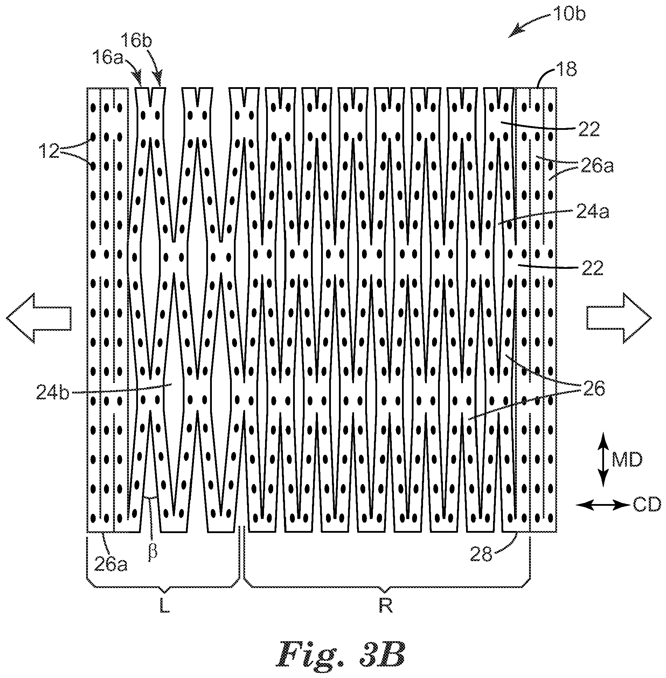

Referring to FIG. 3A and 3B, the particular arrangement of the bridging regions 22, 22a, and 22b can be designed, for example, based on the desired length of the slits and the amount of spreading desired for the multiple strands 26. Various lengths of bridging regions 22, 22a, and 22b may be useful. In some embodiments, any bridging regions 22 in a given interrupted slit 20 have a combined length in the direction of the interrupted slit of up to 50 (in some embodiments, 40, 30, 25, 20, 15, 10, or 5) percent of the length of the slit web in the MD, but generally the combined length in the direction of the interrupted slit is less than 5 percent. In some embodiments, the length of one bridging region in the direction of the interrupted slit is up to 3, 2, or 1.5 mm and at least 0.25, 0.5, or 0.75 mm. In some embodiments, the number of bridging regions along the length of the slit web 10a in the direction of the interrupted slit is up to 1.5, 1.25, 1.0, 0.75, 0.60, or 0.5 per cm. The distance between bridging regions 22 in the direction of the interrupted slit may be, for example, at least 0.75, 1.0, 1.25, 1.5, or 1.75 cm. Furthermore, the length of the interrupted slit portions between bridging regions can be adjusted and may be selected to maximize the distance between bridging regions, for example, to provide the first mechanical fastening portions. In some embodiments, the length of the slit portions 20a, 20b is at least 8 (in some embodiments, at least 10, 12, 14, 15, 16, 17, 18, 19, or 20) mm. In some embodiments, a ratio of a length of the slit portions to a width of one of the multiple strands is at least 2 to 1 (in some embodiments, at least 3:1, 5:1, 10:1, 12.5:1, or 15:1). In slit webs in which a ratio of a length of the slit portions to the width of the multiple strands is less than 2:1, the tensile stress developed in the second direction may become too high to allow the slit web to spread readily.

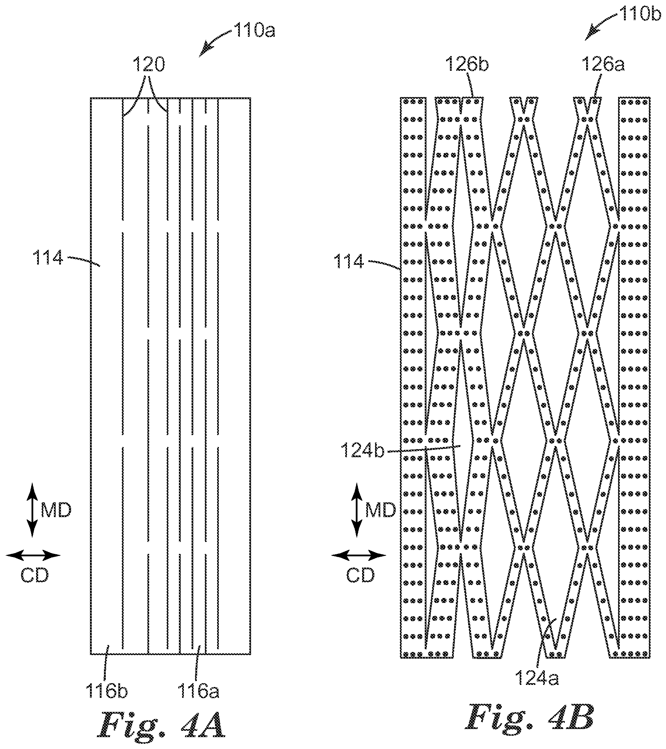

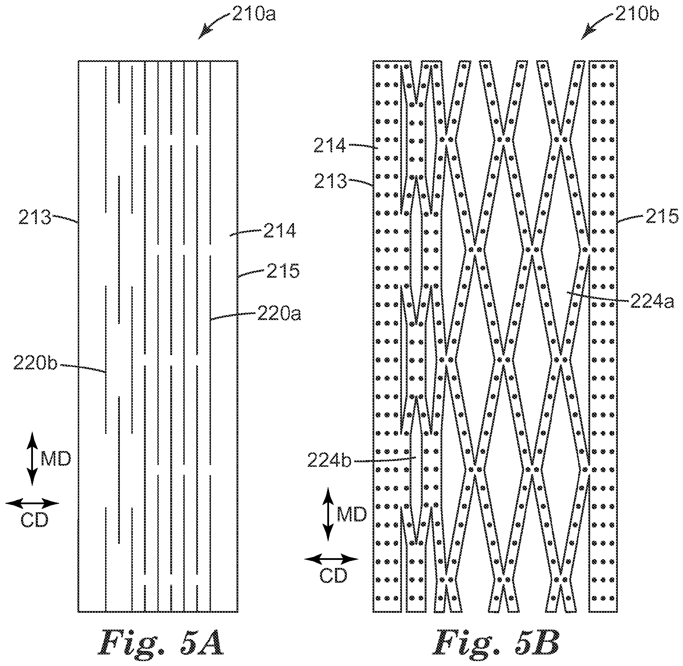

In some embodiments, slit portions 20a, 20b have a regular pattern that repeats down the slit web 10a. In the embodiment illustrated in FIG. 3A, spacing (e.g., in the MD or other direction of the interrupted slits) between slit portions 20a may be uniform or substantially uniform (that is, the spacing may differ by up to 2 percent, 1 percent, or less than 1 or 0.5 percent) although this is not a requirement as described below in connection with the embodiment illustrated in FIGS. 5A and 5B. In the embodiment illustrated in FIG. 3A, the interrupted slits 20 are evenly spaced among the rows of male fastening elements 12 although this is not a requirement as described below in connection with FIGS. 4A and 4B. For multiple rows 16 of male fastening elements 12 that are evenly spaced, as illustrated, the spacing (e.g., distance in the CD in the illustrated embodiment) between multiple rows 16 may differ by up to 10, 5, 2.5, or 1 percent. Likewise, for slits that are evenly spaced, the spacing (e.g., distance in the CD) between the slits may differ by up to 10, 5, 2.5, or 1 percent. In some embodiments, there is a slit between every row or every other row of male fastening elements. For openings that are evenly spaced, the spacing (e.g., distance in the CD) between the openings may differ by up to 10, 5, 2.5, or 1 percent.

FIG. 3B illustrates the effect of spreading the slit web like that shown in FIG. 3A to different extents at different locations. When the slit web 10b is spread in the direction, shown with the arrows, multiple strands 26 of the web are provided, which are attached to each other at least at some of the intact bridging regions 22 and separated from each other between at least some of the intact bridging regions 22. In the illustrated embodiment, the separation between at least some of the multiple strands creates openings 24a and 24b. The formation of openings provides an increase in the width of the slit web (that is, the dimension in the CD). In some embodiments, the width of the spread web is at least 5, 10, 15, 20, or 25 percent greater than the width of the input slit web. In some embodiments, the width of the spread web is up to 40, 50, 75, 100, 150, or 200 percent greater than the width of the input slit web. In FIG. 3B, the left side "L" of the slit web 10b is spread to a greater extent than the right side "R" of the slit web 10b; therefore, openings 24b are larger than openings 24a. The left side "L" of the slit web 10b may be useful for providing first mechanical fastening portions, and the right side "R" of the slit web 10b may be useful for providing second mechanical fastening portions in some embodiments of the fastening laminates disclosed herein.

In the embodiment illustrated in FIG. 3B, at least two strands 26a, including at least two rows of male fastening elements on each edge of the mechanical fastener, are not separated. A spread mechanical fastening web having strands on the edge that are not spread apart may be advantageous in some embodiments, for example, to provide a reticulated mechanical fastening strip or patch with a straight edge or to provide a second mechanical fastening portion without openings. Such a mechanical fastening portion may be easier for a user to open. This can also be accomplished by not slitting the edges of the web.

In spread mechanical fastening web 10b illustrated in FIG. 3B, the male fastening elements 12 on a first strand 26 are arranged in a series 16a that is non-parallel to a series 16b of male fastening elements 12 on a second, adjacent strand 26. The series 16a and 16b of multiple upstanding posts and the multiple strands themselves from which they project can undulate or zig-zag along the length of the spread mechanical fastening web 10b, for example, from the top edge 18 to the bottom edge 28. In the illustrated embodiment, the caps visible on the upstanding posts of the male fastening elements 12 have an oval shape, and these caps are oriented in different directions along the multiple strands 26 in the MD. When the caps are circular in shape, it may not be observed that the caps are oriented in different directions along the multiple strands 26, unless the cap is marked in some way. In the illustrated embodiment, the caps on a first strand 26 are oriented in a different direction than the caps on a second, adjacent strand 26. In embodiments in which slit web 10a includes male fastening elements having loop-engaging overhangs aligned only parallel to the MD, spreading the slit web 10a typically results in the loop-engaging overhangs oriented in different directions along the multiple strands in the MD as shown in FIG. 3B. When loop-engaging overhangs are oriented in multiple directions (e.g., not only one direction such as the machine direction), enhanced engagement of a loop material may advantageously result. These features are also shown in spread mechanical fastening webs 110b and 210b, shown in FIGS. 4B and 5B, and described below.

While FIGS. 3A and 3B illustrate a backing 14 with male fastening elements 12 that comprise upstanding posts, in some embodiments, the slit web can be a web including female fastening elements (that is, a loop material).

FIG. 4A illustrates an example of a slit web portion 110a with interrupted slits 120 in the backing 114 and having male fastening elements (not shown), which is similar to the portion of slit web 10a shown in FIG. 3A. However, in the embodiment shown in FIG. 4A, the distance between interrupted slits 120 in the CD varies from one portion of slit web 110a to the other. For one portion, interrupted slits 120 are more closely spaced and provide narrower strands 116a. In another portion, interrupted slits 120 are spaced further apart and provide wider strands 116b. When slit web 110a is spread as shown in FIG. 4B, narrower strands 126a are attached to each other at intact bridging regions but separated from each other between these bridging regions to provide openings 124a. Similarly, wider strands 126b are attached to each other at intact bridging regions but separated from each other between these bridging regions to provide openings 124b. The openings 124a formed between interrupted slits 120 that are more closely spaced are larger than the openings 124b between interrupted slits 120 that are spaced further apart. Narrower strands 126a may be useful as first mechanical fastening portions, and wider strands 126b may be useful as second mechanical fastening portions in some embodiments of the fastening laminate disclosed herein.

For embodiments of the slit web shown in FIGS. 3A and 4A, the number of slits may be adjusted depending on the desired product. The slits may be evenly spaced as shown in FIG. 3A or unevenly spaced as shown in FIG. 4A as desired. In some embodiments, there are up to 10, 9, 8, 7, 6, 5, 4, 3, 2, or 1 slits per 10 mm across the width of the slit web in the CD. In some embodiments, changing the number of slits across the slit web may be related to the number of rows of male fastening elements 12 between any two adjacent slits, depending on the density of the male fastening elements on the web. The number of rows of male fastening elements between any two adjacent slits may be adjusted depending on the requirements of the application. In some embodiments, there are up to 10, 9, 8, 7, 6, 5, 4, 3, 2 rows, or 1 row of male fastening elements between any two adjacent slits. Typically, the width dimension of each of the multiple strands formed between slits is wider than at least the bases of the upstanding posts of the male fastening elements.

Although FIGS. 4A and 4B illustrate mechanical fasteners with male fastening elements, the same slitting pattern and spreading using the method disclosed herein can be carried out with webs including female fastening elements (that is, loop materials).

In some embodiments, for controlling the ability of a slit web to spread, it is useful to change the lengths of the interrupted slits and/or change the length of the bridging regions in the direction of the interrupted slit. As shown in FIG. 5A, slit portions 220a have different lengths than slit portions 220b, which results in openings 224a and 224b having different sizes after the slit web 210a is spread as shown in FIG. 5B. That is, openings 224a are longer in the MD and wider in the CD than openings 224b. In the embodiment shown in FIGS. 5A and 5B, the length of the interrupted slits varies from one side of slit web 210a to the other, with longer slits 220a closer to one edge 215 and shorter slits 220b closer to the opposite edge 213. Shorter slits are spaced by longer bridging regions in the backing 214 than the longer slits. The slit web 210a near edge 213, including the openings 224b, may be useful as second mechanical fastening portions, and a portion of the slit web nearer edge 215 including openings 224a may be useful as first mechanical fastening portions in some embodiments of the fastening laminate disclosed herein. The slit web 210a and the slit-and-spread web 210b shown in FIG. 5A and 5B, respectively, may include male mechanical fastening elements, as shown in FIG. 5B, or female fastening elements (e.g., loops).

For any of the embodiments of the fastening laminate according to the present disclosure having a backing with openings, the openings can have a variety of geometric shapes. In the illustrated embodiments, the geometric shapes are polygons, which may be quadrilaterals such as rhombuses. In some embodiments, the openings in the backing of the first mechanical fastening portion and/or the second mechanical fastening portion are at least one of rhombus-shaped or hexagonal. In some embodiments, including the embodiment illustrated in FIG. 3B, the multiple strands of the web attached to each other at least at some of the intact bridging regions form an angle β of less than 90 degrees, in some embodiments, less than 60 degrees, 45 degrees, or 20 degrees, and in some embodiments, in a range from 0.5 to 20 degrees. For example, in some embodiments, when a slit web having 19 mm slit portions 20a is spread so that the width of the spread mechanical fastening web is 100% greater than the input slit web, the angle β is typically in a range from about 5 to 10 degrees. In some embodiments, curved lines may be used, which can result in crescent shaped openings after spreading.

Although FIGS. 3A, 4A, and 5A each show interrupted slits 20, 120, 220a, and 220b extending parallel to the MD of the slit web, the interrupted slits may be made in any desired direction. For example, slits may be made at an angle from 1 to 85 degrees to the MD of the slit web. In some embodiments, slits are made at an angle in a range from 35 to 55 degrees (e.g., 45 degrees) to the MD of the slit web. In some embodiments, slits are made in the CD. Any width of the slit web 10a, 110a, and 210a in the CD may be useful. For example, the slit web may have a width in the CD in a range from 5 mm to 10 cm, 1 cm to 5 cm, or 1 cm to 3 cm wide. When it is said that a slit "extends" in a certain direction, it is meant that the slit is arranged or aligned in that direction or at least predominantly in that direction. The slit may be linear. As used herein a "linear" slit can be defined by two points in a line on the web. The slit may also be substantially linear, which means that the slit can have a slight curvature or slight oscillation. Some oscillation or curvature may result, for example, from the process of slitting a continuous web as would be understood by a person skilled in the art. The slits may also be non-linear. For example, they may have a wavy or sawtooth pattern with a small amplitude relative to the width of the web, and such a slit would also be considered to extend in predominantly in a certain direction.

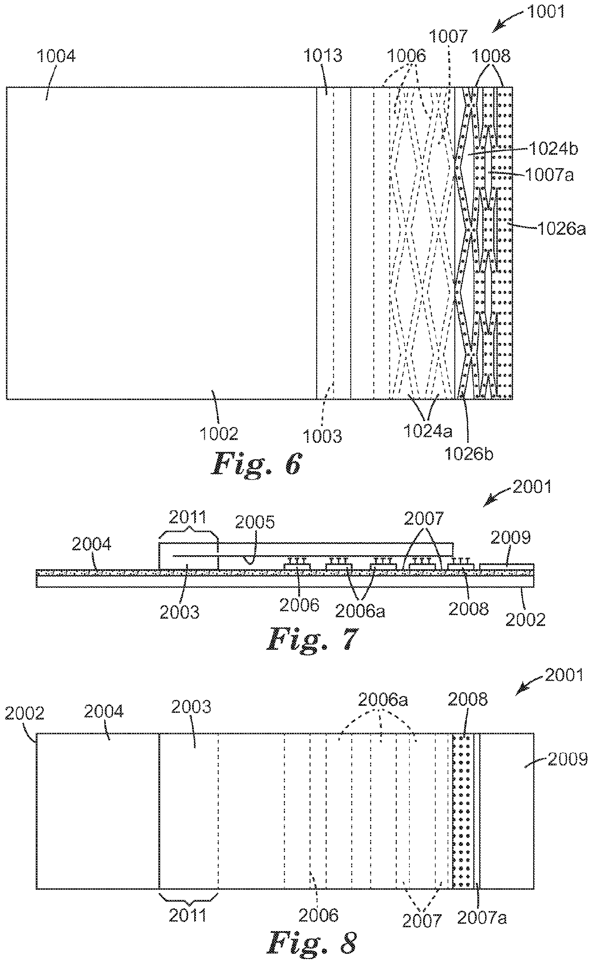

FIG. 6 shows a top view of an embodiment of a fastening laminate according to the present disclosure in which the first and the second mechanical fastening portions include the backing with the openings formed by strands of the backing attached to each other at intact bridging regions in the backing and separated from each other between the bridging regions. In the illustrated embodiment, the first and second mechanical fastening portions are made from a slit and spread web similar to that shown in FIG. 5B. Similar to FIG. 2, FIG. 6 illustrates a fastening laminate as it may appear when it is initially manufactured, before it is installed on an absorbent article. Fastening laminate 1001 includes a substrate 1002. First mechanical fastening portions 1006 and second mechanical fastening portions 1008, both provided by a slit and spread web are adhered to the substrate 1002 using an adhesive layer 1004. The side of the release tape 1003 visible in the top view can be coated with an adhesive (not shown), which allows it to be attached to the substrate 1002 with a strip 1013. The strip 1013 is attached to the adhesive (not shown) on the release tape 1003 and the adhesive 1004 on the substrate in order to attach the release tape 1003 to the substrate 1002. The adhesive (not shown) on one side of the release tape also allows it to be attached to an absorbent article in a Y-bond configuration. The opposite side of the release tape 1003 has a release surface (not visible in FIG. 6). The release tape 1003 covers at least some of the first mechanical fastening portions 1006 and first portions of the adhesive layer 1007 in openings 1024a. The first mechanical portions, covered by the release surface, are shown in phantom. The release tape 1003 does not cover the second mechanical fastening portions 1008, and the adhesive in the openings 1024b is exposed. In the illustrated embodiment, both strands 1026a and 1026b can be considered second mechanical fastening portions. It is easily visible in this top view that the ratio of the surface area of the uncovered second mechanical fastening portions 1008 to adhesive surface area 1007a is larger than the ratio of the surface area of the covered first mechanical fastening portions 1006 to adhesive surface area 1007 covered by the release surface. Also, in the embodiment illustrated in FIG. 6, at least some of the second openings 1024b are smaller than first openings 1024a.

Slit webs typically develop little to no stress in the direction of spreading at least upon initially straining the slit web in the spreading direction. The stress can be affected by the size of the slits in a slit web. The slits provide regions where any means for transmission of force in the spreading direction is substantially absent. Accordingly, for any of the embodiments of the fastening laminate according to the present disclosure in which the first and/or the second mechanical fastening portions include the backing with openings formed by strands of the backing attached to each other at intact bridging regions in the backing and separated from each other between the bridging regions, spreading the slit mechanical fastener to provide multiple strands separated from each other between at least some of the bridging regions to provide openings can be carried out in a variety of suitable ways. When spreading is desired in the machine direction of a continuous web (e.g., with interrupted slits are made in the cross-web direction), monoaxial spreading in the machine direction can be performed by propelling the thermoplastic web over rolls of increasing speed, with the downweb roll speed faster than the upweb roll speed. When spreading is desired in a cross-direction or other angle to the machine direction, spreading can be carried out on a continuous web using a flat film tenter apparatus, diverging rails, diverging disks, or a series of bowed rollers. A method of using diverging disks for spreading a slit web is described in Int. Pat. App. Pub. No. WO 2013/172957 (Gilbert et al.). When the first and/or second mechanical fastening portion in the laminates disclosed herein are provided by a mechanical fastening patch cut to a desired size, spreading the slit mechanical fastener may also be carried out, for example, by hand.

In a web process for providing first and/or the second mechanical fastening portions include the backing with openings formed by strands of the backing attached to each other at intact bridging regions in the backing and separated from each other between the bridging regions, it may be useful to spread a slit web in the cross-machine direction by moving the slit web over a crowned surface. A crowned surface can be considered any forming surface that lengthens the path of a portion of the slit web. Useful crowned surfaces have a varying height dimension in a direction corresponding to the CD of the slit web. Generally, the height of the crowned surface is greatest at its center. The crowned surface may be a smooth surface having a generally spherical or elliptical shape in which the diameter or axis continuously increases toward its center, but a useful crowned surface need not have a uniform height variation over its entire portion that contacts the slit web. For example, the crown surface may have a flat portion where the slit web first contacts it, and the curvature of the crowned surface in a direction corresponding to the CD of the slit web may increase in the direction of the slit web path. The crowned surface may also have, in some embodiments, ridges or other surface irregularities.

Any surface over which a web in tension is bent or wrapped around is believed to impart a force on the web that is normal or perpendicular to the web. Because of the varying height of a crowned surface, the force imparted on a web by a crowned surface is not evenly distributed. Without wanting to be bound by theory, it is believed that the crowned surface can spread open a slit web as described herein because a component of the normal force generated by a crowned surface will be in the cross-web direction. The cross-direction strength of the web is relatively low because of the slits in the web, and the amount of tension in the web that would resist spreading is low. Therefore, the cross-directional component of the force generated by a crowned surface can induce spreading of a slit web.

The amount of spreading that can result by moving a slit web over a crowned surface can be limited by the frictional force resisting the cross-directional movement of the spreading web. Because of this, it may be desirable to minimize the friction between the slit web and the crowned surface. Such friction can be decreased if at least a portion of the crowned surface is a low-friction surface. For example, a least a portion of the crowned surface can be made from a low-friction material or can be coated with a low-friction coating. Also, if the crowned surface is an air bearing, friction between the slit web and the crowned surface may be decreased. Since the coefficient of kinetic friction of two materials is generally lower than the corresponding coefficient of static friction, it is typically desirable that the crowned surface and the slit web are not moving at the same speed in the same direction so that the crowned surface and the slit web can have a "slipping" interface. Accordingly, in some cases, the crowned surface does not rotate, or, in other words, it is stationary. Further information regarding a spreading a slit web using a crowned surface can be found in Int. Pat. App. Pub. No. WO 2013/172960 (Gilbert et al.).

Another web process for providing first and/or the second mechanical fastening portions having the backing with openings formed by strands of the backing attached to each other at intact bridging regions in the backing and separated from each other between the bridging regions includes running a slit web in the machine direction onto a stretchable surface. The slit web is in contact with the stretchable surface for a path length in the machine direction, and for at least a portion of the path length, the stretchable surface is stretching in the cross-machine direction. The traction between the slit web and the stretchable surface during the stretching at least partially separates at least some of the multiple strands of the slit web in a second direction transverse to the first direction. In some cases, the slit web is run over a roller comprising two rotating diverging disks that are laterally spaced and have the stretchable surface between them that stretches in the cross-machine direction for a portion of a rotation of the two rotating diverging disks. The stretchable surface stretches for 180 degrees of the rotation of the diverging disks as it moves from the position where the disks are closest together to the position where the disks are farthest apart. The band then retracts for 180 degrees of the rotation of the diverging disks as it moves from the position where the disks are farthest apart to the position where the disks are closest together. Multiple strands of a slit web that come into contact with the stretchable surface at any position where the stretchable surface is stretching will be spread apart in the direction of the stretch. The slit web may be positioned to be in contact with the stretchable surface for any portion of the rotation sufficient to at least partially separate at least some of the multiple strands of the slit web. The angle of the diverging disks can be selected depending on the desired amount of spread in the slit web. For example, each diverging disk may independently be angled at least 1, 2, 3, 4, or 5 degrees and up to 20, 15, or 10 degrees with respect to the machine direction of the running web. In some embodiments, each diverging disk is independently angled in a range from 1 to 10 degrees or 2.5 to 7.5 degrees. Since the diverging disks may be independently angled, the method according to the present disclosure may be useful for spreading the slit web uniformly or non-uniformly with respect to the center of the slit web. In some embodiments, the strands closer to one edge of the slit web may be spread apart more than the strands closer to the opposite edge of the slit web, for example, as shown in FIG. 3B.

A variety of materials can be useful as stretchable surfaces. For examples, elastic bands, elastic tubing, coiled springs, or an elastic sleeve may be useful and may be attached to the diverging disks by pins, clamps, belts, or any other of a variety of useful methods. The term "elastic" refers to any material (such as a film that is 0.002 mm to 0.5 mm thick) that exhibits recovery from stretching or deformation. In some embodiments, a material may be considered to be elastic if, upon application of a stretching force, it can be stretched to a length that is at least about 25 (in some embodiments, 50) percent larger than its initial length and can recover at least 40, 50, 60, 70, 80, or 90 percent of its elongation upon release of the stretching force. Typically, elastic materials are considered "high-friction" materials and may allow for sufficient traction between the slit web and the stretching surface so that the slit web spreads apart along with the stretching surface. A particular elastic material may be selected for the stretching surface to maximize the traction with the slit web. It may also be useful to increase the traction between a given stretching surface and a slit web, for example, by increasing the machine direction tension on the web or by providing the stretching surface with surface structure (e.g., microstructures). In embodiments in which the slit web includes male fastening elements comprising upstanding posts having bases attached to the slit web, the upstanding posts can be directed to face against the stretching surface, which may be a structured surface, to increase traction. When the stretching surface is a coiled spring, the coiled spring may be metal (e.g., aluminum or steel) and may be coated with a high-friction coating, if desired. The high-friction coating can be, for example, a coating of an elastomeric material or a plasma coating known to provide a high-friction surface. Different types of stretchable surfaces may be useful together to provide different advantageous properties, for example, elongation, stiffness, or friction properties. Further information regarding a spreading a slit web using a stretchable surface can be found in copending U.S. Pat. App. Serial No. 13/891,287 (Rothwell et al.), filed on May 10, 2013.

When separating strands of a web including interrupted slits, it is typically advantageous to not allow the attached multiple strands of the spread web to twist out-of-plane. Twisted strands of the spread web create a non-uniform contact surface, which can complicate heat transfer to the web and complicate the use of a nip in further web processing (e.g., annealing or laminating as described below) since the twisted strands may be crushed by the nip. In a web process, tension applied in the machine direction, which exerts a force normal to the slit web, can be adjusted to keep the attached strands in plane. Also, adjusting the amount of spreading (e.g., using the geometry of a crowned surface or the angle of diverging disks) can also control out-of-plane twisting.

In some embodiments, the first and second mechanical fastening portions in the form of a spread mechanical fastening web is annealed before it is attached to the substrate with the adhesive layer. In some embodiments, annealing comprises heating the spread web. In some embodiments, annealing comprises heating and then cooling (e.g., rapidly cooling) the spread web to maintain its configuration. Heating and/or annealing can be carried out, for example, after the spread web has been spread to the final desired extent or at an intermediate stage, for example, if the spread web is spread a second time with a second stretchable surface. Annealing the spread web can be useful, for example, depending on the extent of spreading, and can be useful to maintain the openings between multiple strands, for example, when the width of the slit web has been increased by at least 50 percent. Annealing can also be useful, for example, for maintaining at least some of the multiple strands in a substantially coplanar arrangement. In some embodiments, heating is only applied to the second surface of the spread mechanical fastening web (i.e., the surface opposite the first surface from which the mechanical fastening elements project) to minimize any damage to the mechanical fastening elements that may result from heating. Heating may be accomplished, for example, using heated rollers after the slit web is spread. Non-contact heating methods such as IR irradiation, hot air treatment, or by directing the web through a heated chamber may also be useful. It may also be useful, in some embodiments, to heat the slit web using any of these heating methods before it is spread.

Whether or not (or before or after) a spread mechanical fastening web is annealed, the spread web may be handled by a high-friction roller (e.g., comprising an elastomeric material as described above or a material with a rough surface). The high-friction roller may be heated or chilled, if desired, or may be useful at room temperature. A high-friction roller may be useful, for example, for holding the spread web in a spread configuration whether or not the web is annealed. In some embodiments, a heated, high-friction roller may be useful for annealing the spread web.

In some cases, the spread mechanical fastening web may be in the form of a roll. The bridging regions interrupting the interrupted slits allow the spread web to be handled as an integral unit, for example, to be handled in roll form and converted as desired, for example, before it is laminated to the substrate.

In some embodiments of the fastening laminate according to the present disclosure, the first mechanical fastening portion and second mechanical fastening portion are discrete strips of a backing with upstanding fastening elements. In other words, the first and second mechanical fastening portions are not attached to each other. In some embodiments, discrete first and second mechanical fastening strips are attached to an adhesive layer through their second major surfaces (i.e., opposite the first major surfaces having the upstanding posts) in a parallel fashion at a distance from each other such that an alternating sequence of multiple parallel strips of exposed adhesive layer and of the mechanical fastening strips is obtained. Such an assembly is described, for example, in U.S. Pat. Appl. Pub. No. 2007/0039142 (Petersen et al.). The configuration of the first and second mechanical fastening strips may be as described, for example, in Int. Pat. Appl. Pub. No. WO2011/163020 (Hauschildt et al.). The first and second mechanical fastening strips may be evenly spaced on the adhesive layer. As an example, FIGS. 7 and 8 illustrate a fastening laminate 2001 as it may appear when it is initially manufactured. Fastening laminate 2001 includes a substrate 2002. A layer of adhesive 2004 is provided on the substrate 2002. First mechanical fastening portions 2006, 2006a and second mechanical fastening portion 2008 are adhered to the substrate 2002 using the adhesive layer 2004. Film 2009, useful as a fingerlift, is also adhered to the substrate 2002 using the adhesive layer 2004. Near the manufacturing end of the fastening laminate 2001, a first end portion 2011 of the release tape 2003 is attached to substrate 2002. The release tape 2003 has a release surface 2005. In FIG. 7, the release tape 2003 is shown folded back on itself at the fold line, and the release tape covers first mechanical portions 2006, 2006a and the first portions 2007 of the adhesive layer 2004 between them but does not cover the second mechanical fastening portion 2008. In FIG. 8, the discrete first mechanical portions 2006, 2006a, which are covered by the release tape 2003, are shown in phantom. Fastening laminate 2001 also includes a second portion 2007a of the adhesive layer not covered by the second mechanical fastening portion 2008. When the fastening laminate is installed on an absorbent article, the second portion 2007a of the adhesive layer is therefore advantageously available to engage the fibers of the absorbent article, for example, to prevent the fastening laminate from popping open. However, typically the surface area of the second portion of the adhesive layer 2007a is small enough such that it can be released from the absorbent article without requiring excessive force. As clearly shown in FIG. 8, a first ratio of a surface area of the first mechanical fastening portions 2006, 2006a to a surface area of the first portions 2007 of the adhesive layer is smaller than a second ratio of a surface area of the second mechanical fastening portion 2008 to a surface area of the second portion 2007a of the adhesive layer.

Although FIGS. 7 and 8 illustrate five discrete strips of mechanical fastener, other numbers of strips may be useful. For example, the fastening laminate may have two, three, or four discrete strips. In some embodiments, it is desirable to have three discrete strips of mechanical fastener. In these embodiments, at least one discrete strip is completely covered by the release tape. In some of these embodiments, two discrete strips are completely covered by the release tape. In some of these embodiments, two discrete strips are completely covered by the release tape, and a third discrete strip is partially covered by the release tape. In other of these embodiments, a center discrete strip is partially uncovered by the release tape. In yet other of these embodiments, two of the discrete strips are completely uncovered by the release tape.

In some embodiments, the second adhesive portions, formed either by openings or by separation of the discrete strips may have a width in the separation direction (e.g., CD) of up to 1.5 mm, 1 mm, or 0.75 mm. These second adhesive portions may have a width in the separation direction of greater than 0.5 mm or at least 0.6 mm, 0.7 mm, 0.75 mm, or 0.8 mm. The width of these second adhesive portions may be in a range from 0.6mm, 0.7 mm, or 0.75 mm up to 1.5 mm. In some embodiments, the first adhesive portions, formed either by openings or by separation of the discrete strips may have a width in the separation direction (e.g., CD) of greater than 1.5 mm, for example, at least 2 mm or at least 3 mm. For openings that have varying widths, these values refer to the maximum width.

Providing slits in a mechanical fastening web, whether the slits are interrupted slits to leave the mechanical fastening web intact or full slits to provide discrete strips of mechanical fastener, can be carried out in a variety of ways. For example, rotary die cutting of a continuous web may be useful. Interrupted slits can be made, for example, by using rotary cutting blades having gaps to form the bridging regions. The height of the blade in the gaps may be adjusted to allow for the bridging regions to be partially cut or not cut at all, depending on the desired embodiment. Other cutting methods (e.g., laser cutting) may also be useful. Cutting can be performed from either surface of the continuous web. A slit may be cut "through" the web, which means that the slit cuts through the entire thickness of the web. In other embodiments, the slit may be a partial-depth slit can be readily pulled apart. The partial-depth slit may penetrate, for example, 80, 85, or 90 percent of the thickness of the web or more, which means the solution to the equation:

$$(\text{depth of the slit divided by the thickness of the web}) \times 100$$

is at least 80, 85, or 90 in some embodiments. Other methods of slitting a web can be found, for example, in U.S. Pat. Appl. Pub. No. 2011/0313389 (Wood et al.).

The first and second mechanical fastening portions, whether they include multiple strands of a backing attached to each other at intact bridging regions and separated from each other between at least some of the bridging regions to provide openings or whether they are discrete strips of a backing with upstanding fastening elements, are attached to the substrate using the adhesive layer. In some embodiments, it can be advantageous to adhere the first and second mechanical fastening portions to the substrate immediately after they are spread apart to the desired degree. When the first and second mechanical fastening portions include multiple strands of a backing attached to each other at intact bridging regions that are directed onto an adhesive-coated substrate soon after at least some of the multiple strands are at least partially separated, annealing and/or directing the spread web onto a high-friction surface may not be necessary since the adhesive can keep the strands separated. In some embodiments, adhesive lamination is carried out in a nip formed by two rollers. In some embodiments, joining first and second mechanical fastening portions to the adhesive-coated substrate is not carried out in a nip. Instead, in some embodiments, the laminate is nipped downweb from where the first and second mechanical fastening portions are joined to the adhesive-coated substrate. This method may help to keep the laminate flat.

The substrate 2, 1002, 2002, may be continuous (i.e., without any through-penetrating holes) or discontinuous (e.g. comprising through-penetrating perforations or pores). The substrate 2, 1002, 2002 may comprise a variety of suitable materials including woven webs, non-woven webs (e.g., spunbond webs, spunlaced webs, airlaid webs, meltblown web, and bonded carded webs), textiles, plastic films (e.g., single- or multilayered films, coextruded films, laterally laminated films, or films comprising foam layers), and combinations thereof. Combinations can include multi-layer substrates having coextensive layers of different materials or lateral laminates of different materials. In some embodiments, the substrate 2, 1002, 2002 is a fibrous material (e.g., a woven, nonwoven, or knit material). The term "non-woven" refers to a material having a structure of individual fibers or threads that are interlaid but not in an identifiable manner such as in a knitted fabric. In some embodiments, the carrier comprises multiple layers of nonwoven materials with, for example, at least one layer of a meltblown nonwoven and at least one layer of a spunbonded nonwoven, or any other suitable combination of nonwoven materials. For example, the substrate 2, 1002, 2002 may be a spunbond-meltbond-spunbond, spunbond-spunbond, or spunbond-spunbond-spunbond multilayer material. Or, the substrate 2, 1002, 2002 may be a composite web comprising a nonwoven layer and a dense film layer. Fibrous materials that may provide useful substrates 2, 1002, 2002 may be made from natural fibers (e.g., wood or cotton fibers), synthetic fibers (e.g., thermoplastic fibers), or a combination of natural and synthetic fibers. Examples of suitable materials for forming thermoplastic fibers include polyolefins (e.g., polyethylene, polypropylene, polybutylene, ethylene copolymers, propylene copolymers, butylene copolymers, and copolymers and blends of these polymers), polyesters, and polyamides. The fibers may also be multi-component fibers, for example, having a core of one thermoplastic material and a sheath of another thermoplastic material. Useful substrates 2, 1002, 2002 may have any suitable basis weight or thickness that is desired for a particular application. For a fibrous substrate, the basis weight may range, e.g., from at least about 5, 8, 10, 20, 30, or 40 grams per square meter, up to about 400, 200, or 100 grams per square meter. The substrate may be up to about 5 mm, about 2 mm, or about 1 mm in thickness and/or at least about 0.1, about 0.2, or about 0.5 mm in thickness.

In some embodiments of the fastening laminate according to the present disclosure, one or more zones of the substrate 2, 1002, 2002 may comprise one or more elastically extensible materials extending in at least one direction when a force is applied and returning to approximately their original dimension after the force is removed. However, in some embodiments, at least the portion of the substrate 2, 1002, 2002 joined to the first and second mechanical fastening portions is not stretchable. In some embodiments, the portion of substrate 2, 1002, 2002 joined to the multiple strands will have up to a 10 (in some embodiments, up to 9, 8, 7, 6, or 5) percent elongation in the CD. In some embodiments, the substrate may be extensible but nonelastic. In other words, the substrate may have an elongation of at least 5, 10, 15, 20, 25, 30, 40, or 50 percent but substantially no recovery from the elongation (e.g., less than or equal to 10 or 5 percent recovery). Suitable extensible substrates may include nonwovens (e.g., spunbond, spunbond meltblown spunbond, or carded nonwovens). In some embodiments, the nonwoven may be a high elongation carded nonwoven (e.g., HEC). In some embodiments, the substrate is not pleated.

In some embodiments of the fastening laminate according to the present disclosure, the first and/or second mechanical fastening portion 6, 6a, 8, 1006, 1008, 2006, 2006a, 2008 is made of a thermoplastic material. Suitable thermoplastic materials include polyolefin homopolymers such as polyethylene and polypropylene, copolymers of ethylene, propylene and/or butylene; copolymers containing ethylene such as ethylene vinyl acetate and ethylene acrylic acid; polyesters such as poly(ethylene terephthalate), polyethylene butyrate and polyethylene napthalate; polyamides such as poly(hexamethylene adipamide); polyurethanes; polycarbonates; poly(vinyl alcohol); ketones such as polyetheretherketone; polyphenylene sulfide; and mixtures thereof. Typically, in embodiments in which the first and/or second mechanical fastening portion includes male fastening elements, the thermoplastic is a polyolefin (e.g., polyethylene, polypropylene, polybutylene, ethylene copolymers, propylene copolymers, butylene copolymers, and copolymers and blends of these materials).

In some embodiments of the first and/or second mechanical fastening portion 6, 6a, 8, 1006, 1008, 2006, 2006a, 2008 useful in the fastening laminate disclosed herein, the backing and the male fastening elements are typically integral (that is, formed at the same time as a unit, unitary). Upstanding posts on a backing can be made, for example, by feeding a thermoplastic material onto a continuously moving mold surface with cavities having the inverse shape of the posts. The thermoplastic material can be passed between a nip formed by two rolls or a nip between a die face and roll surface, with at least one of the rolls having the cavities. The cavities may be in the inverse shape of a capped post having a loop-engaging head or may be in the inverse shape of an upstanding post without loop-engaging heads (e.g., a precursor to a male fastening element). Pressure provided by the nip forces the resin into the cavities. In some embodiments, a vacuum can be used to evacuate the cavities for easier filling of the cavities. The nip typically has a large enough gap such that a coherent backing is formed over the cavities. The mold surface and cavities can optionally be air or water cooled before stripping the integrally formed backing and upstanding hook elements from the mold surface such as by a stripper roll. If the posts formed upon exiting the cavities do not have loop-engaging heads, loop-engaging heads could be subsequently formed into hooks by a capping method as described in U.S. Pat. No. 5,077,870 (Melbye et al.). Typically, the capping method includes deforming the tip portions of the hook elements using heat and/or pressure. The heat and pressure, if both are used, could be applied sequentially or simultaneously.

In some embodiments, when a thermoplastic material is fed onto a continuously moving mold surface with cavities having the inverse shape of upstanding posts, slitting the web and spreading the slit web according to the method disclosed herein can be carried out before or after a capping step is carried out to form loop-engaging heads. Also, deforming the distal tip to form a cap can be carried out, for example, after slitting through the web but before spreading the slit web; after spreading the slit web but before annealing; or after annealing as desired. The formation of male fastening elements can also include a step in which the shape of the cap is changed, for example, as described in U.S. Pat. No. 6,132,660 (Kampfer). Such a cap modifying step can be carried out directly after capping or after any of the slitting, spreading, or further processing steps described herein.

Suitable tool rolls include those formed from a series of plates defining a plurality of post-forming cavities about its periphery such as those described, for example, in U.S. Pat. No. 4,775,310 (Fischer). Cavities may be formed in the plates by drilling or photoresist technology, for example. Other suitable tool rolls may include wire-wrapped rolls, which are disclosed along with their method of manufacturing, for example, in U.S. Pat. No. 6,190,594 (Gorman et al.). Another example of a method for forming a thermoplastic backing with upstanding posts includes using a flexible mold belt defining an array of upstanding post-shaped cavities as described in U.S. Pat. No. 7,214,334 (Jens et al.). Yet other useful methods for forming a thermoplastic backing with upstanding posts can be found in U.S. Pat. Nos. 6,287,665 (Hammer), 7,198,743 (Tuma), and 6,627,133 (Tuma).

The male fastening elements in first and/or second mechanical fastening portion 6, 6a, 8, 1006, 1008, 2006, 2006a, 2008 disclosed herein may have loop-engaging heads that have an overhang or may be upstanding posts having distal tips that can be formed into loop-engaging heads, if desired. The term "loop-engaging" as used herein relates to the ability of a male fastening element to be mechanically attached to a loop material. Generally, male fastening elements with loop-engaging heads have a head shape that is different from the shape of the post. For example, the male fastening element may be in the shape of a mushroom (e.g., with a circular or oval head enlarged with respect to the stem), a hook, a palm-tree, a nail, a T, or a J. The loop-engageability of male fastening elements may be determined and defined by using standard woven, nonwoven, or knit materials. A region of male fastening elements with loop-engaging heads generally will provide, in combination with a loop material, at least one of a higher peel strength, higher dynamic shear strength, or higher dynamic friction than a region of posts without loop-engaging heads. Male fastening elements that have "loop-engaging overhangs" or "loop-engaging heads" do not include ribs that are precursors to fastening elements (e.g., elongate ribs that are profile extruded and subsequently cut to form male fastening elements upon stretching in the direction of the ribs). Such ribs would not be able to engage loops before they are cut and stretched. Such ribs would also not be considered upstanding posts. Typically, male fastening elements that have loop-engaging heads have a maximum thickness dimension (in either dimension normal to the height) of up to about 1 (in some embodiments, 0.9, 0.8, 0.7, 0.6, 0.5, or 0.45) millimeter.

In some embodiments, the thickness of the backing of the first and/or second mechanical fastening portions 6, 6a, 8, 1006, 1008, 2006, 2006a, 2008 described herein may be up to about 400, 250, 150, 100, 75 or 50 micrometers, depending on the desired application, which does not necessarily include the height of male or female mechanical fastening elements on the surface of the web. In some embodiments, the thickness of the backing is in a range from 30 to about 225 micrometers, from about 50 to about 200 micrometers, or from about 100 to about 150 micrometers. In some embodiments, the backing has stretch-induced molecular orientation, for example, when the thermoplastic web is stretched after formation of upstanding posts. In embodiments in which multiple strands of the spread web are attached to each other at intact bridging regions and separated from each other between the intact bridging regions, there may be some stress-induced orientation localized in the bridging regions. However, there may not be any macroscopic stretch-induced molecular orientation in the direction of the slits or in the direction of spreading.

In some embodiments, the male fastening elements have a maximum height (above the backing) of up to 3mm, 1.5 mm, 1 mm, or 0.5 mm and, in some embodiments a minimum height of at least 0.05 mm, 0.1 mm, or 0.2 mm. In some embodiments, the upstanding posts have aspect ratio (that is, a ratio of height to width at the widest point) of at least about 2:1, 3:1, or 4:1.

Loop materials useful for practicing some embodiments of the present disclosure (e.g., when the first and/or second mechanical fastening portion 6, 6a, 8, 1006, 1008, 2006, 2006a, 2008 is a loop material) can be any suitable material that interlocks with corresponding hook fastening elements. In some embodiments, the loop fastening elements are typically formed from knitted fabrics, woven fabrics, or non-woven fabrics. The term "non-woven" refers to a material having a structure of individual fibers or threads that are interlaid but not in an identifiable manner such as in a knitted fabric. Examples of non-woven webs include spunbond webs, spunlaced webs, airlaid webs, meltblown web, and bonded carded webs. The spread web may include fiber loops projecting from a knitted, woven, or non-woven backing or may be extrusion-bonded, adhesive-bonded, and/or sonically-bonded fiber loops. Useful loop materials may be made of natural fibers (e.g., wood or cotton fibers), synthetic fibers (e.g., thermoplastic fibers), or a combination of natural and synthetic fibers. Examples of suitable materials for forming thermoplastic fibers include polyolefins (e.g., polyethylene, polypropylene, polybutylene, ethylene copolymers, propylene copolymers, butylene copolymers, and copolymers and blends of these polymers), polyesters, and polyamides. The fibers may also be multi-component fibers, for example, having a core of one thermoplastic material and a sheath of another thermoplastic material.

In some embodiments, the loop material using for practicing some embodiments of the present disclosure comprises a fibrous layer disposed on a backing. Suitable backings include textiles, paper, thermoplastic films (e.g., single- or multilayered films, coextruded films, laterally laminated films, or films comprising foam layers), and combinations thereof. For thermoplastic backings, the thermoplastic can be any of those described above in connection with a thermoplastic backing having male fastening elements. Examples of suitable loop materials are described, for example, in U.S. Pat. Nos. 5,256,231 (Gorman et al.) and 5,389,416 (Mody et al.). As described in U.S. Pat. No. 5,256,231 (Gorman et al.), the fibrous layer in a loop material according to some embodiments can comprise arcuate portions projecting in the same direction from spaced anchor portions on the backing.

The adhesive layer 4, 1004, 2004 in any of the embodiments of the fastening laminate according to the present disclosure is generally made up of an adhesive having a peel strength that is sufficient to permanently attach the substrate 2, 1002, 2002 to the outside surface of an absorbent article and to permanently attach the first and second mechanical fastening portions 6, 6a, 8, 1006, 1008, 2006, 2006a, 2008 to the substrate 2, 1002, 2002. The adhesive used may be any conventional adhesive, including pressure sensitive adhesives (PSAs) and non-pressure sensitive adhesives. PSAs are well known to those of ordinary skill in the art to possess properties including the following: (1) aggressive and permanent tack, (2) adherence with no more than finger pressure, (3) sufficient ability to hold onto an adherend, and (4) sufficient cohesive strength to be cleanly removable from the adherend. Materials that have been found to function well as PSAs are polymers designed and formulated to exhibit the requisite viscoelastic properties resulting in a desired balance of tack, peel adhesion, and shear holding power. Suitable pressure sensitive adhesives include acrylic resin and natural or synthetic rubber-based adhesives and may be hot melt pressure sensitive adhesives. Illustrative rubber based adhesives include styrene-isoprene-styrene, styrene-butadiene-styrene, styrene-ethylene/butylenes-styrene, and styrene-ethylene/propylene-styrene that may optionally contain diblock components such as styrene isoprene and styrene butadiene. The adhesive may be applied using hot-melt, solvent, or emulsion techniques.

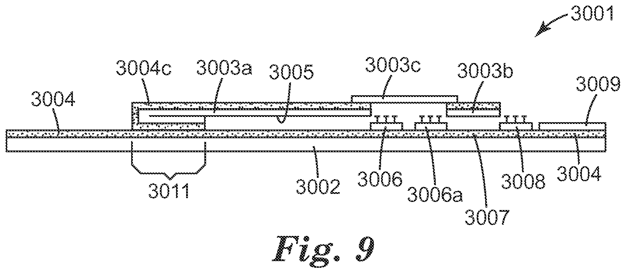

Release tape 3, 1003, 2003 can be a thermoplastic film, prepared, for example, from any of the thermoplastics described above for first and second mechanical fastening portions that include male fastening elements. Suitable release tapes can include single- or multilayered films, coextruded films, laterally laminated films, and combinations thereof. The release tape includes a release surface 5, 2005, which can be a release coating (e.g., a silicone, fluorochemical, or carbamate coating) on the thermoplastic film. A structured surface can also be useful as a release surface. In some embodiments, the release tape is a unitary piece of thermoplastic film. In other embodiments, the release tape can include multiple materials joined together. In some embodiments, a portion of the release tape is a woven web, nonwoven web (e.g., spunbond webs, spunlaced webs, airlaid webs, meltblown web, and bonded carded webs), or textile. In some embodiments, a portion of the release tape is a nonwoven web. For example, a piece of nonwoven web can be laterally laminated to two pieces of release coated thermoplastic film as shown in FIG. 9.

Referring now to FIG. 9, fastening laminate 3001, first substrate 3002 has an adhesive layer 3004 disposed thereon. First and second mechanical fastening portions 3006, 3006a, and 3008 and film 3009, useful as a fingerlift, are positioned on the adhesive layer 3004. The release tape includes three portions: film portions 3003a and 3003b and nonwoven portion 3003c. The film portions 3003a and 3003b have a release surface 3005, in the form of a release coating, and an adhesive coating 3004a on the opposite surface. The nonwoven portion 3003c has a release surface in the sense that the nonwoven can releasably engage with first mechanical fastening portions 3006, 3006a. The adhesive layer 3004c allows the release tape to be attached to an absorbent article and adheres the nonwoven portion 3003c to film portions 3003a and 3003b. A portion of the release tape 3011 is attached to the substrate 3002 through an adhesive-to-adhesive connection. The release tape is folded back on itself, and the release surface 3005 covers the exposed adhesive layer 3004 including the first portion 3007 of the adhesive layer while the nonwoven portion 3003c is positioned to cover first mechanical fastening portions 3006, 3006a. Second mechanical fastening portion 3008 is not covered by the release tape.

While in many embodiments, the release tape 3, 1003, 2003 is coated with an adhesive layer on a surface opposite the release surface, this is not a requirement. For example, the release tape may be attached to the substrate with the adhesive layer on the substrate and attached to the edge of an absorbent article using another bonding method (e.g., ultrasonic bonding). In some embodiments, the release tape is folded back on itself, for example, as shown in FIGS. 1, 2, 7, 8, and 9. During manufacturing, the release tape is typically applied to the substrate in a pre-folded condition although it is possible in some cases to fold the release tape after attaching one end to the substrate. As shown in FIG. 6, the release tape may also be attached to the substrate using a separate strip or patch. The strip or patch can be made from a material such as any of those described above for the substrate. When the release tape 3, 1003, 2003 is coated with an adhesive layer on a surface opposite the release surface, the strip or patch can adhere to both the release tape and the substrate to connect them. Otherwise, other bonding methods (e.g., ultrasonic bonding) may be used. Any of the release tape, the pre-folded release tape, or the separate strip for attaching the release tape to the substrate can be provided in roll form.

Fastening laminates according to the present disclosure are useful, for example, in absorbent articles. Absorbent articles may have at least a front waist region, a rear waist region, and a longitudinal center line bisecting the front waist region and the rear waist region, wherein at least one of the front waist region or the rear waist region comprises the fastening laminate disclosed herein. The fastening laminate may be in the form of a fastening tab that is bonded to at least one of the front waist region or the rear waist region. A fastening tab may extend outwardly from at least one of the left longitudinal edge or the right longitudinal edge of the absorbent article. In other embodiments, the fastening laminate may be an integral ear portion of the absorbent article.

In absorbent articles according to the present disclosure, the topsheet is typically permeable to liquid and designed to contact a wearer's skin, and the outwardly facing backsheet is typically impermeable to liquids. There is typically an absorbent core encased between the topsheet and the backsheet. Various materials can be useful for the topsheet, the backsheet, and the absorbent core in an absorbent article according to the present disclosure. Examples of materials useful for topsheets include apertured plastic films, woven fabrics, nonwoven webs, porous foams, and reticulated foams. In some embodiments, the topsheet is a nonwoven material. Examples of suitable nonwoven materials include spunbond or meltblown webs of fiber forming polymer filaments (e.g., polyolefin, polyester, or polyamide filaments) and bonded carded webs of natural polymers (e.g., rayon or cotton fibers) and/or synthetic polymers (e.g., polypropylene or polyester fibers). The nonwoven web can be surface treated with a surfactant or otherwise processed to impart the desired level of wettability and hydrophilicity. The backsheet is sometimes referred to as the outer cover and is the farthest layer from the user. The backsheet functions to prevent body exudates contained in absorbent core from wetting or soiling the wearer's clothing, bedding, or other materials contacting the diaper. The backsheet can be a thermoplastic film (e.g., a poly(ethylene) film). The thermoplastic film may be embossed and/or matte finished to provide a more aesthetically pleasing appearance. The backsheet can also include woven or nonwoven fibrous webs, for example, laminated to the thermoplastic films or constructed or treated to impart a desired level of liquid impermeability even in the absence of a thermoplastic film. Suitable backsheets also include vapor or gas permeable microporous "breathable" materials that are substantially impermeable to liquid. Suitable absorbent cores include natural, synthetic, or modified natural polymers that can absorb and hold liquids (e.g., aqueous liquids). Such polymers can be crosslinked (e.g., by physical entanglement, crystalline domains, covalent bonds, ionic complexes and associations, hydrophilic associations such as hydrogen bonding, and hydrophobic associations or Van der Waals forces) to render them water insoluble but swellable. Such absorbent materials are usually designed to quickly absorb liquids and hold them, usually without release. Examples of suitable absorbent materials useful in absorbent articles disclosed herein include wood pulp or other cellulosic materials and super absorbent polymers (SAP).

The openings or spaces between the first and second mechanical fastening portions can provide breathability and flexibility to fastening laminate, which may enhance the comfort of the wearer, for example, of an absorbent article. The mechanical fastener also is typically able to cover a relatively large area with a relatively small amount of material, which may lower its cost. Also, because of the large area that may be covered by the mechanical fastener and the combination of mechanical and adhesive fastening, the mechanical fastener may provide performance enhancement in an absorbent article, for example, by resist shifting forces such as torsional or rotational forces caused by movement of the wearer of the absorbent article. For example, in use, fitting an absorbent article such as a diaper about the wearer usually requires the front and back waist portions of the diaper to overlap each other. As the diaper is worn the movements of the wearer tend to cause the overlapping front and back waist portions to shift position relative to each other. Unless such shifting is limited, the fit and containment characteristics of the diaper may be degraded as the diaper is worn. A fastening laminate according to the present disclosure may provide improved fit and closure stability by resisting such shifting because of its relatively larger area, dual fastening mechanism, and flexibility.

When the laminate is prepared in a continuous process or formed into a roll, the web laminate or roll is typically cut in a cross direction to form a fastening tab, for example, for an absorbent article. The resulting fastening tab can be attached to the absorbent article by attaching the substrate and a second surface of the release tape, opposite the release surface, of the fastening tab along one edge of an absorbent article using any of the methods described above. The absorbent article can then be packaged in a storage configuration, with the first portion of the adhesive layer adhered to the release tape, and with the second mechanical fastening portion and any second portion of the adhesive layer in contact with an inside surface of the absorbent article.

Mechanical fasteners and laminates made according to the present disclosure may also be useful in many other fastening applications, for example, assembly of automotive parts or any other application in which releasable attachment may be desirable.

**Claims**

1. A fastening laminate (1, 1001, 3001) comprising:

   a substrate (2, 1002, 3002) with an adhesive layer (4, 1004, 3004) disposed thereon;
   a mechanical fastener (6, 6a, 8, 8a, 1006, 1008, 3006, 3008) on the adhesive layer (4, 1004, 3004), wherein the mechanical fastener (6, 6a, 8, 8a, 1006, 1008, 3006, 3008) includes a first opening (24a, 1024a) that reveals a first portion of the adhesive layer (7, 1007, 3007); and
   a release tape (3, 1003, 3003a, 3003b, 3003c) having a release surface (5, 3005), the release tape (3, 1003, 3003a, 3003b, 3003c) having an end portion (11, 1013, 3011) attached to the substrate (2, 1002, 3002) closer to a first portion of the mechanical fastener (6, 6a, 1006, 3006) than a second portion of the mechanical fastener (8, 8a, 1008, 3008), wherein the release surface (5, 3005) can cover the first portion of the mechanical fastener (6, 6a, 1006, 3006) and at least partially cover the first portion of the adhesive layer (7, 1007, 3007), but cannot completely cover the second portion of the mechanical fastener (8, 8a, 1008, 3008), and wherein the first portion of the adhesive layer (7, 1007, 3007) is between the first portion of the mechanical fastener (6, 6a, 1006, 3006) and the second portion of the mechanical fastener (8, 8a, 1008, 3008).

2. The fastening laminate (1, 1001, 3001) of claim 1, further comprising a second portion of the adhesive layer (7a, 1007a) not covered by the second portion of the mechanical fastener (8, 8a, 1008, 3008), wherein the first (7, 1007) and second portions of the adhesive layer (7a, 1007a) are on opposite sides of the second portion of the mechanical fastener (8, 8a, 1008, 3008).

3. The fastening laminate (1, 1001, 3001) of claim 2, wherein a first ratio of a surface area of the first portion of the mechanical fastener (6, 6a, 1006, 3006) to a surface area of the first portion of the adhesive layer (7, 1007) is smaller than a second ratio of a surface area of the second portion of the mechanical fastener (8, 8a, 1008, 3008) to a surface area of the second portion of the adhesive layer (7a, 1007a).

4. The fastening laminate (1, 1001, 3001) of any one of claims 1 to 3, wherein the first portion of the mechanical fastener (6, 6a, 1006, 3006) is provided by a backing (14, 114, 214) with upstanding fastening elements (12), wherein the backing (14, 114, 214) has the first opening (24a, 124a, 224a, 1024a) that reveals the first portion of the adhesive layer (7, 1007).

5. The fastening laminate (1, 1001, 3001) of claim 4, wherein the first portion of the mechanical fastener (6, 6a, 1006, 3006) is one of multiple first mechanical fastening portions (6, 6a, 1006, 3006) provided by strands (26, 126a, 126b, 1026a) of the backing (14, 114, 214) attached to each other at intact bridging regions (22) in the backing (14, 114, 214) and separated from each other between the intact bridging regions (22) to form multiple first openings (24a, 124a, 224a, 1024a) that reveal multiple first portions of the adhesive layer (7, 1007) not covered by the first mechanical fastening portions (6, 6a, 1006, 3006).

6. The fastening laminate (1, 1001, 3001) of claim 5, wherein the second portion of the mechanical fastener (8, 8a, 1008, 3008) is one of multiple second mechanical fastening portions (8, 8a, 1008, 3008) provided by strands (26, 126a, 126b, 1026a) of the backing (14, 114, 214) attached to each other at intact bridging regions (22) in the backing (14, 114, 214) and separated from each other between the intact bridging regions (22) to form second openings (24b, 124b, 224b, 1024b) that reveal multiple second portions of the adhesive layer (7a, 1007a) not covered by the second mechanical fastening portions (8, 8a, 1008, 3008), and wherein the second openings (24b, 124b, 224b, 1024b) between the second mechanical fastening portions (8, 8a, 1008, 3008) (8, 8a, 1008, 3008) are smaller than the first openings (24a, 124a, 224a, 1024a) between the first mechanical fastening portions (6, 6a, 1006, 3006).

7. The fastening laminate (1, 1001, 3001) of claim 4, wherein the second portion (8, 8a, 1008, 3008) of the mechanical fastener is provided by the backing (14, 114, 214) with upstanding fastening elements (12), wherein the backing (14, 114, 214) has a second opening (24b, 124b, 224b, 1024b) that reveals a second portion of the adhesive layer (7a, 1007a), and wherein the first opening (24a, 124a, 224a, 1024a) is larger than the second opening (24b, 124b, 224b, 1024b).

8. The fastening laminate (1, 1001, 3001) of claim 6 or 7, wherein the first and second openings (24a, 24b, 124a, 124b, 224a, 224b, 1024a, 1024b) are at least one of diamond-shaped or hexagonal.

9. An absorbent article (100) comprising the fastening laminate (1, 1001, 3001) of any one of claims 1 to 8, wherein the substrate (2, 1002, 3002) and a second surface of the release tape (3, 1003, 3003a, 3003b, 3003c), opposite the release surface (5, 3005), are attached along one edge of the absorbent article.

10. The absorbent article (100) of claim 9, wherein the absorbent article (100) is in a storage configuration, with the first portion of the adhesive layer (7, 1007) adhered to the release tape (3, 1003, 3003a, 3003b, 3003c), and with the second mechanical fastening portion (8, 8a, 1008, 3008) and any second portion of the adhesive layer (7a, 1007a) in contact with an inside surface of the absorbent article (100).

11. A method of making a fastening laminate (1, 1001, 3001), the method comprising:

> providing an adhesive layer (4, 1004, 3004) on a substrate (2, 1002, 3002);
> attaching a mechanical fastener (6, 6a, 8, 8a, 1006, 1008, 3006, 3008) to the adhesive layer (4, 1004, 3004), wherein the mechanical fastener (6, 6a, 8, 8a, 1006, 1008, 3006, 3008) includes a first opening (24a, 1024a) that reveals a first portion of the adhesive layer (7, 1007, 3007); and
> attaching a release tape (3, 1003, 3003a, 3003b, 3003c) to the adhesive layer (4, 1004, 3004) closer to a first portion of the mechanical fastener (6, 6a, 1006, 3006) than a second portion of the mechanical fastener (8, 8a, 1008, 3008), the release tape (3, 1003, 3003a, 3003b, 3003c) having a release surface (5, 3005), wherein the release surface (5, 3005) covers the first portion of the mechanical fastener (6, 6a, 1006, 3006), at least partially covers the first portion of the adhesive layer (7, 1007, 3007), but does not completely cover the second portion of the mechanical fastener (8, 8a, 1008, 3008), and wherein the first portion of the adhesive layer (7, 1007, 3007) is between the first portion of the mechanical fastener (6, 6a, 1006, 3006) and the second portion of the mechanical fastener (8, 8a, 1008, 3008).

**12.** The method of claim 11, further comprising

slitting through a thermoplastic backing (14, 114, 214) having upstanding posts (12) to provide a slit mechanical fastener (10a, 110a, 210a) having interrupted slits (20a, 20b, 120, 220, 220b), wherein each interrupted slit (20a, 20b, 120, 220, 220b) is interrupted by at least one intact bridging region (22) of the slit mechanical fastener (10a, 110a, 210a); and

spreading the slit mechanical fastener (10a, 110a, 210a) to provide strands (26, 126a, 126b, 1026a) of the mechanical fastener (10b, 110b, 210b) attached to each other at least at some of the bridging regions (22) and separated from each other between at least some of the bridging regions (22) to provide openings (24a, 24b, 124a, 124b, 224a, 224b, 1024a, 1024b);

wherein attaching the mechanical fastener (6, 6a, 8, 8a, 1006, 1008, 3006, 3008) to the adhesive layer (4, 1004, 3004) comprises attaching the strands (26, 126a, 126b, 1026a) of the slit mechanical fastener (10b, 110b, 210b) in a spread configuration to maintain the openings (24a, 24b, 124a, 124b, 224a, 224b, 1024a, 1024b) between the strands (26, 126a, 126b, 1026a), and wherein at least one of the strands (26, 126a, 126b, 1026a) provides the first portion of the mechanical fastener (6, 6a, 1006, 3006) and at least one of the openings (24a, 124a, 224a, 1024a) reveals the first portion of the adhesive layer (7, 1007, 3007).

**13.** The method of claim 12, wherein at least one of the following is true: a distance between at least some of the interrupted slits (20a, 20b, 120, 220, 220b) varies; a size of the intact bridging regions (22) varies between at least some of the interrupted slits (20a, 20b, 120, 220, 220b), or the slit mechanical fastener (10b, 110b, 210b) is spread non-uniformly so that at least some of the openings (24a, 24b, 124a, 124b, 224a, 224b, 1024a, 1024b) are not uniformly sized.

**14.** The method of claim 12 or 13, wherein the first portion of the mechanical fastener (6, 6a, 1006, 3006) is one of multiple first mechanical fastening portions (6, 6a, 1006, 3006) provided by strands (26, 126a, 126b, 1026a) of the backing (14, 114, 214) attached to each other at intact bridging regions (22) in the backing (14, 114, 214) and separated from each other between the bridging regions (22) to form multiple first openings (24a, 124a, 224a, 1024a) that reveal multiple first portions of the adhesive layer (7, 1007) not covered by the first mechanical fastening portions (6, 6a, 1006, 3006).

**15.** The method of any one of claims 12 to 14, wherein the second portion (8, 8a, 1008, 3008) of the mechanical fastener is one of multiple second mechanical fastening portions (8, 8a, 1008, 3008) provided by strands (26, 126a, 126b, 1026a) of the backing (14, 114, 214) attached to each other at intact bridging regions (22) in the backing (14, 114, 214) and separated from each other between the intact bridging regions (22) to form second openings (24b, 124b, 224b, 1024b) that reveal multiple second portions of the adhesive layer (7a, 1007a) not covered by the second mechanical fastening portions (8, 8a, 1008, 3008), and wherein the second openings (24b, 124b, 224b, 1024b) between the second mechanical fastening portions (8, 8a, 1008, 3008) are smaller than the first openings (24a, 124a, 224a, 1024a) between the first mechanical fastening portions (6, 6a, 1006, 3006).

**Patentansprüche**

**1.** Befestigungslaminat (1, 1001, 3001), aufweisend:

ein Substrat (2, 1002, 3002) mit einer darauf aufgebrachten Klebeschicht (4, 1004, 3004);

ein mechanisches Befestigungsmittel (6, 6a, 8, 8a, 1006, 1008, 3006, 3008) auf der Klebeschicht (4, 1004, 3004), wobei das mechanische Befestigungsmittel (6, 6a, 8, 8a, 1006, 1008, 3006, 3008) eine erste Öffnung (24a, 1024a) aufweist, die einen ersten Abschnitt der Klebeschicht (7, 1007, 3007) freigibt; und

ein Trennband (3, 1003, 3003a, 3003b, 3003c) mit einer Trennfläche (5, 3005), wobei das Trennband (3, 1003, 3003a, 3003b, 3003c) einen Endabschnitt (11, 1013, 3011) aufweist, der an dem Substrat (2, 1002, 3002) näher an einem ersten Abschnitt des mechanischen Befestigungsmittels (6, 6a, 1006, 3006) als an einem zweiten Abschnitt des mechanischen Befestigungsmittels (8, 8a, 1008, 3008) befestigt ist, wobei die Trennfläche (5, 3005) den ersten Abschnitt des mechanischen Befestigungsmittels (6, 6a, 1006, 3006) abdecken kann und zumindest teilweise den ersten Abschnitt der Klebeschicht (7, 1007, 3007) abdecken kann, aber den zweiten Abschnitt des mechanischen Befestigungsmittels (8, 8a, 1008, 3008) nicht vollständig abdecken kann, und wobei sich der erste Abschnitt der Klebeschicht (7, 1007, 3007) zwischen dem ersten Abschnitt des mechanischen Befestigungsmittels (6, 6a, 1006, 3006) und dem zweiten Abschnitt des mechanischen Befestigungsmittels (8, 8a, 1008, 3008) befindet.

2. Befestigungslaminat (1, 1001, 3001) nach Anspruch 1, ferner aufweisend einen zweiten Abschnitt der Klebeschicht (7a, 1007a), der nicht durch den zweiten Abschnitt des mechanischen Befestigungsmittels (8, 8a, 1008, 3008) abgedeckt ist, wobei sich der erste (7, 1007) und der zweite Abschnitt der Klebeschicht (7a, 1007a) auf zueinander entgegengesetzten Seiten des zweiten Abschnitts des mechanischen Befestigungsmittels (8, 8a, 1008, 3008) befinden.

3. Befestigungslaminat (1, 1001, 3001) nach Anspruch 2, wobei ein erstes Verhältnis einer Oberfläche des ersten Abschnitts des mechanischen Befestigungsmittels (6, 6a, 1006, 3006) zu einer Oberfläche des ersten Abschnitts der Klebeschicht (7, 1007) kleiner ist als ein zweites Verhältnis einer Oberfläche des zweiten Abschnitts des mechanischen Befestigungsmittels (8, 8a, 1008, 3008) zu einer Oberfläche des zweiten Abschnitts der Klebeschicht (7a, 1007a).

4. Befestigungslaminat (1, 1001, 3001) nach einem der Ansprüche 1 bis 3, wobei der erste Abschnitt des mechanischen Befestigungsmittels (6, 6a, 1006, 3006) durch einen Träger (14, 114, 214) mit aufrechten Befestigungselementen (12) bereitgestellt wird, wobei der Träger (14, 114, 214) die erste Öffnung (24a, 124a, 224a, 1024a) aufweist, die den ersten Abschnitt der Klebeschicht (7, 1007) freigibt.

5. Befestigungslaminat (1, 1001, 3001) nach Anspruch 4, wobei der erste Abschnitt des mechanischen Befestigungsmittels (6, 6a, 1006, 3006) einer von mehreren ersten mechanischen Befestigungsabschnitten (6, 6a, 1006, 3006) ist, die durch Stränge (26, 126a, 126b, 1026a) des Trägers (14, 114, 214) bereitgestellt werden, die an intakten Überbrückungsbereichen (22) im Träger (14, 114, 214) aneinander angebracht sind und zwischen den intakten Überbrückungsbereichen (22) voneinander getrennt sind, um mehrere erste Öffnungen (24a, 124a, 224a, 1024a) zu bilden, die mehrere erste Abschnitte der Klebeschicht (7, 1007) freigeben, die nicht durch die ersten mechanischen Befestigungsabschnitte (6, 6a, 1006, 3006) abgedeckt sind.

6. Befestigungslaminat (1, 1001, 3001) nach Anspruch 5, wobei der zweite Abschnitt des mechanischen Befestigungsmittels (8, 8a, 1008, 3008) einer von mehreren zweiten mechanischen Befestigungsabschnitten (8, 8a, 1008, 3008) ist, die durch Stränge (26, 126a, 126b, 1026a) des Trägers (14, 114, 214) bereitgestellt werden, die an intakten Überbrückungsbereichen (22) im Träger (14, 114, 214) aneinander angebracht und zwischen den intakten Überbrückungsbereichen (22) voneinander getrennt sind, um zweite Öffnungen (24b, 124b, 224b, 1024b) zu bilden, die mehrere zweite Abschnitte der Klebeschicht (7a, 1007a) freigeben, die nicht durch die zweiten mechanischen Befestigungsabschnitte (8, 8a, 1008, 3008) abgedeckt sind, und wobei die zweiten Öffnungen (24b, 124b, 224b, 1024b) zwischen den zweiten mechanischen Befestigungsabschnitten (8, 8a, 1008, 3008) (8, 8a, 1008, 3008) kleiner sind als die ersten Öffnungen (24a, 124a, 224a, 1024a) zwischen den ersten mechanischen Befestigungsabschnitten (6, 6a, 1006, 3006).

7. Befestigungslaminat (1, 1001, 3001) nach Anspruch 4, wobei der zweite Abschnitt (8, 8a, 1008, 3008) des mechanischen Befestigungsmittels durch den Träger (14, 114, 214) mit aufrechten Befestigungselementen (12) bereitgestellt wird, wobei der Träger (14, 114, 214) eine zweite Öffnung (24b, 124b, 224b, 1024b) aufweist, die einen zweiten Abschnitt der Klebeschicht (7a, 1007a) freigibt, und wobei die erste Öffnung (24a, 124a, 224a, 1024a) größer ist als die zweite Öffnung (24b, 124b, 224b, 1024b).

8. Befestigungslaminat (1, 1001, 3001) nach Anspruch 6 oder 7, wobei die erste und zweite Öffnung (24a, 24b, 124a, 124b, 224a, 224b, 1024a, 1024b) rautenförmig und/oder sechseckig sind.

9. Absorbierender Artikel (100), der das Befestigungslaminat (1, 1001, 3001) nach einem der Ansprüche 1 bis 8 aufweist, wobei das Substrat (2, 1002, 3002) und eine zweite Oberfläche des Trennbandes (3, 1003, 3003a, 3003b, 3003c), die der Trennfläche (5, 3005) entgegengesetzt ist, entlang eines Rands des absorbierenden Artikels angebracht sind.

10. Absorbierender Artikel (100) nach Anspruch 9, wobei der absorbierende Artikel (100) in einer Verstaukonfiguration vorliegt, wobei der erste Abschnitt der Klebeschicht (7, 1007) auf das Trennband (3, 1003, 3003a, 3003b, 3003c) geklebt ist und der zweite mechanische Befestigungsabschnitt (8, 8a, 1008, 3008) und jeder zweite Abschnitt der Klebeschicht (7a, 1007a) mit einer Innenfläche des absorbierenden Artikels (100) in Kontakt steht.

11. Verfahren zum Herstellen eines Befestigungslaminats (1, 1001, 3001), wobei das Verfahren Folgendes aufweist:

Bereitstellen einer Klebeschicht (4, 1004, 3004) auf einem Substrat (2, 1002, 3002);

Anbringen eines mechanischen Befestigungsmittels (6, 6a, 8, 8a, 1006, 1008, 3006, 3008) an der Klebeschicht (4, 1004, 3004), wobei das mechanische Befestigungsmittel (6, 6a, 8, 8a, 1006, 1008, 3006, 3008) eine erste Öffnung (24a, 1024a) aufweist, die einen ersten Abschnitt der Klebeschicht (7, 1007, 3007) freilegt; und Anbringen eines Trennbandes (3, 1003, 3003a, 3003b, 3003c) an der Klebeschicht (4, 1004, 3004) näher an einem ersten Abschnitt des mechanischen Befestigungsmittels (6, 6a, 1006, 3006) als an einem zweiten Abschnitt des mechanischen Befestigungsmittels (8, 8a, 1008, 3008), wobei das Trennband (3, 1003, 3003a, 3003b, 3003c) eine Trennfläche (5, 3005) aufweist, wobei die Trennfläche (5, 3005) den ersten Abschnitt des mechanischen Befestigungsmittels (6, 6a, 1006, 3006) abdeckt, zumindest teilweise den ersten Abschnitt der Klebeschicht (7, 1007, 3007) abdeckt, aber den zweiten Abschnitt des mechanischen Befestigungsmittels (8, 8a, 1008, 3008) nicht vollständig abdeckt, und wobei sich der erste Abschnitt der Klebeschicht (7, 1007, 3007) zwischen dem ersten Abschnitt des mechanischen Befestigungsmittels (6, 6a, 1006, 3006) und dem zweiten Abschnitt des mechanischen Befestigungsmittels (8, 8a, 1008, 3008) befindet.

12. Verfahren nach Anspruch 11, ferner aufweisend

Durchschlitzen eines thermoplastischen Trägers (14, 114, 214) mit aufrechten Pfosten (12) zum Bereitstellen eines geschlitzten mechanischen Befestigungsmittels (10a, 110a, 210a) mit unterbrochenen Schlitzen (20a, 20b, 120, 220, 220b), wobei jeder unterbrochene Schlitz (20a, 20b, 120, 220, 220b) durch mindestens einen intakten Überbrückungsbereich (22) des geschlitzten mechanischen Befestigungsmittels (10a, 110a, 210a) unterbrochen ist; und Spreizen des geschlitzten mechanischen Befestigungsmittels (10a, 110a, 210a), um Stränge (26, 126a, 126b, 1026a) des mechanischen Befestigungsmittels (10b, 110b, 210b) bereitzustellen, die mindestens an einigen der Überbrückungsbereiche (22) aneinander angebracht und zwischen mindestens einigen der Überbrückungsbereiche (22) getrennt sind, um Öffnungen (24a, 24b, 124a, 124b, 224a, 224b, 1024a, 1024b) bereitzustellen; wobei das Anbringen des mechanischen Befestigungsmittels (6, 6a, 8, 8a, 1006, 1008, 3006, 3008) an der Klebeschicht (4, 1004, 3004) ein Anbringen der Stränge (26, 126a, 126b, 1026a) des geschlitzten mechanischen Befestigungsmittels (10b, 110b, 210b) in einer gespreizten Konfiguration zum Beibehalten der Öffnungen (24a, 24b, 124a, 124b, 224a, 224b, 1024a, 1024b) zwischen den Strängen (26, 126a, 126b, 1026a) aufweist, und wobei mindestens einer der Stränge (26, 126a, 126b, 1026a) den ersten Abschnitt des mechanischen Befestigungsmittels (6, 6a, 1006, 3006) bereitstellt und mindestens eine der Öffnungen (24a, 124a, 224a, 1024a) den ersten Abschnitt der Klebeschicht (7, 1007, 3007) freigibt.

13. Verfahren nach Anspruch 12, wobei mindestens eines der Folgenden zutrifft: ein Abstand zwischen mindestens einigen der unterbrochenen Schlitze (20a, 20b, 120, 220, 220b) variiert; eine Größe der intakten Überbrückungsbereiche (22) zwischen mindestens einigen der unterbrochenen Schlitze (20a, 20b, 120, 220, 220b) variiert, oder das geschlitzte mechanische Befestigungsmittel (10b, 110b, 210b) ist nicht gleichförmig gespreizt, sodass mindestens einige der Öffnungen (24a, 24b, 124a, 124b, 224a, 224b, 1024a, 1024b) nicht gleichförmig bemessen sind.

14. Verfahren nach Anspruch 12 oder 13, wobei der erste Abschnitt des mechanischen Befestigungsmittels (6, 6a, 1006, 3006) einer von mehreren ersten mechanischen Befestigungsabschnitten (6, 6a, 1006, 3006) ist, die durch Stränge (26, 126a, 126b, 1026a) des Trägers (14, 114, 214) bereitgestellt werden, die an intakten Überbrückungsbereichen (22) im Träger (14, 114, 214) aneinander angebracht, und zwischen den Überbrückungsbereichen (22) voneinander getrennt sind, um mehrere erste Öffnungen (24a, 124a, 224a, 1024a) zu bilden, die mehrere erste Abschnitte der Klebeschicht (7, 1007) freigeben, die nicht durch die ersten mechanischen Befestigungsabschnitte (6, 6a, 1006, 3006) abgedeckt sind.

15. Verfahren nach einem der Ansprüche 12 bis 14, wobei der zweite Abschnitt (8, 8a, 1008, 3008) des mechanischen Befestigungsmittels einer von mehreren zweiten mechanischen Befestigungsabschnitten (8, 8a, 1008, 3008) ist, die durch Stränge (26, 126a, 126b, 1026a) des Trägers (14, 114, 214) bereitgestellt werden, die an intakten Überbrückungsbereichen (22) im Träger (14, 114, 214) aneinander angebracht und zwischen den intakten Überbrückungsbereichen (22) voneinander getrennt sind, um zweite Öffnungen (24b, 124b, 224b, 1024b) zu bilden, die mehrere zweite Abschnitte der Klebeschicht (7a, 1007a) freigeben, die nicht durch die zweiten mechanischen Befestigungsabschnitte (8, 8a, 1008, 3008) abgedeckt sind, und wobei die zweiten Öffnungen (24b, 124b, 224b, 1024b) zwischen den zweiten mechanischen Befestigungsabschnitten (8, 8a, 1008, 3008) kleiner sind als die ersten Öffnungen (24a, 124a, 224a, 1024a) zwischen den ersten mechanischen Befestigungsabschnitten (6, 6a, 1006, 3006).

**Revendications**

1.  Stratifié de fixation (1, 1001, 3001) comprenant :

    un substrat (2, 1002, 3002) avec une couche adhésive (4, 1004, 3004) disposée sur celui-ci ;
    une fixation mécanique (6, 6a, 8, 8a, 1006, 1008, 3006, 3008) sur la couche adhésive (4, 1004, 3004), dans lequel la fixation mécanique (6, 6a, 8, 8a, 1006, 1008, 3006, 3008) inclut une première ouverture (24a, 1024a) qui révèle une première partie de la couche adhésive (7, 1007, 3007) ; et
    un ruban de libération (3, 1003, 3003a, 3003b, 3003c) possédant une surface de libération (5, 3005), le ruban de libération (3, 1003, 3003a, 3003b, 3003c) possédant une partie d'extrémité (11, 1013, 3011) fixée au substrat (2, 1002, 3002) plus proche d'une première partie de la fixation mécanique (6, 6a, 1006, 3006) que d'une deuxième partie de la fixation mécanique (8, 8a, 1008, 3008), dans lequel la surface de libération (5, 3005) peut couvrir la première partie de la fixation mécanique (6, 6a, 1006, 3006) et couvrir au moins partiellement la première partie de la couche adhésive (7, 1007, 3007), mais ne peut pas couvrir complètement la deuxième partie de la fixation mécanique (8, 8a, 1008, 3008), et dans lequel la première partie de la couche adhésive (7, 1007, 3007) se trouve entre la première partie de la fixation mécanique (6, 6a, 1006, 3006) et la deuxième partie de la fixation mécanique (8, 8a, 1008, 3008).

2.  Stratifié de fixation (1, 1001, 3001) selon la revendication 1, comprenant en outre une deuxième partie de la couche adhésive (7a, 1007a) non couverte par la deuxième partie de la fixation mécanique (8, 8a, 1008, 3008), dans lequel les première (7, 1007) et deuxième parties de la couche adhésive (7a, 1007a) sont sur des côtés opposés de la deuxième partie de la fixation mécanique (8, 8a, 1008, 3008).

3.  Stratifié de fixation (1, 1001, 3001) selon la revendication 2, dans lequel un premier rapport d'une superficie de la première partie de la fixation mécanique (6, 6a, 1006, 3006) à une superficie de la première partie de la couche adhésive (7, 1007) est inférieur à un deuxième rapport d'une superficie de la deuxième partie de la fixation mécanique (8, 8a, 1008, 3008) à une superficie de la deuxième partie de la couche adhésive (7a, 1007a).

4.  Stratifié de fixation (1, 1001, 3001) selon l'une quelconque des revendications 1 à 3, dans lequel la première partie de la fixation mécanique (6, 6a, 1006, 3006) est fournie par un support (14, 114, 214) avec des éléments de fixation (12) faisant saillie, dans lequel le support (14, 114, 214) comporte la première ouverture (24a, 124a, 224a, 1024a) qui révèle la première partie de la couche adhésive (7, 1007).

5.  Stratifié de fixation (1, 1001, 3001) selon la revendication 4, dans lequel la première partie de la fixation mécanique (6, 6a, 1006, 3006) est une de plusieurs premières parties de la fixation mécanique (6, 6a, 1006, 3006) fournies par des brins (26, 126a, 126b, 1026a) du support (14, 114, 214) attachés les uns aux autres au niveau de régions de liaison intactes (22) dans le support (14, 114, 214) et séparés les uns des autres entre les régions de liaison intactes (22) de façon à former plusieurs premières ouvertures (24a, 124a, 224a, 1024a) qui révèlent plusieurs premières parties de la couche adhésive (7, 1007) non couvertes par les premières parties de la fixation mécanique (6, 6a, 1006, 3006).

6.  Stratifié de fixation (1, 1001, 3001) selon la revendication 5, dans lequel la deuxième partie de la fixation mécanique (8, 8a, 1008, 3008) est une de plusieurs deuxièmes parties de la fixation mécanique (8, 8a, 1008, 3008) fournies par des brins (26,126a, 126b, 1026a) du support (14 114, 214) attachés les uns aux autres au niveau de régions de liaison intactes (22) dans le support (14, 114, 214) et séparés les uns des autres entre les régions de liaison intactes (22) de façon à former des deuxièmes ouvertures (24b, 124b, 224b, 1024b) qui révèlent plusieurs deuxièmes parties de la couche adhésive (7a, 1007a) non couvertes par les deuxièmes parties de la fixation mécanique (8, 8a, 1008, 3008), et dans lequel les deuxièmes ouvertures (24b, 124b, 224b, 1024b) entre les deuxièmes parties de la fixation mécanique (8, 8a, 1008, 3008) sont plus petites que les premières ouvertures (24a, 124a, 224a, 1024a) entre les premières parties de la fixation mécanique (6, 6a, 1006, 3006).

7.  Stratifié de fixation (1, 1001, 3001) selon la revendication 4, dans lequel la deuxième partie (8, 8a, 1008, 3008) de la fixation mécanique est fournie par le support (14, 114, 214) avec des éléments de fixation (12) faisant saillie, dans lequel le support (14, 114, 214) comporte une deuxième ouverture (24b, 124b, 224b, 1024b) qui révèle une deuxième partie de la couche adhésive (7a, 1007a), et dans lequel la première ouverture (24a, 124a, 224a, 1024a) est plus grande que la deuxième ouverture (24b, 124b, 224b, 1024b).

8.  Stratifié de fixation (1, 1001, 3001) selon la revendication 6 ou 7, dans lequel les première et deuxièmes ouvertures (24a, 24b, 124a, 124b, 224a, 224b, 1024a, 1024b) sont au moins l'une d'en forme de losange ou hexagonale.

9. Article absorbant (100) comprenant le stratifié de fixation (1, 1001, 3001) selon l'une quelconque des revendications 1 à 8, dans lequel le substrat (2, 1002, 3002) et une deuxième surface du ruban de libération (3, 1003, 3003a, 3003b, 3003c), opposée à la surface de libération (5, 3005), sont fixés le long d'un bord de l'article absorbant.

10. Article absorbant (100) selon la revendication 9, dans lequel l'article absorbant (100) se présente dans une configuration de stockage, avec la première partie de la couche adhésive (7, 1007) collée au ruban de libération (3, 1003, 3003a, 3003b, 3003c), et avec la deuxième partie de la fixation mécanique (8, 8a, 1008, 3008) et toute deuxième partie de la couche adhésive (7a, 1007a) en contact avec une surface intérieure de l'article absorbant (100).

11. Procédé de fabrication d'un stratifié de fixation (1, 1001, 3001), le procédé comprenant :

   la fourniture d'une couche adhésive (4, 1004, 3004) sur un substrat (2, 1002, 3002) ;
   la fixation d'une fixation mécanique (6, 6a, 8, 8a, 1006, 1008, 3006, 3008) sur la couche adhésive (4, 1004, 3004), dans lequel la fixation mécanique (6, 6a, 8, 8a, 1006, 1008, 3006, 3008) inclut une première ouverture (24a, 1024a) qui révèle une première partie de la couche adhésive (7, 1007, 3007) ; et
   la fixation d'un ruban de libération (3, 1003, 3003a, 3003b, 3003c) sur la couche adhésive (4, 1004, 3004) plus proche d'une première partie de la fixation mécanique (6, 6a, 1006, 3006) qu'une deuxième partie de la fixation mécanique (8, 8a, 1008, 3008), le ruban de libération (3, 1003, 3003a, 3003b, 3003c) possédant une surface de libération (5, 3005), dans lequel la surface de libération (5, 3005) couvre la première partie de la fixation mécanique (6, 6a, 1006, 3006), couvre au moins partiellement la première partie de la couche adhésive (7, 1007, 3007), mais ne couvre pas complètement la deuxième partie de la fixation mécanique (8, 8a, 1008, 3008), et dans lequel la première partie de la couche adhésive (7, 1007, 3007) se trouve entre la première partie de la fixation mécanique (6, 6a, 1006, 3006) et la deuxième partie de la fixation mécanique (8, 8a, 1008, 3008).

12. Procédé selon la revendication 11, comprenant en outre
   la découpe d'un support thermoplastique (14 114, 214) ayant des montants (12) faisant saillie pour produire une fixation mécanique fendue (10a, 110a, 210a) ayant des fentes interrompues (20a, 20b, 120, 220, 220b), dans lequel chaque fente interrompue (20a, 20b, 120, 220, 220b) est interrompue par au moins une zone de liaison intacte (22) de la fixation mécanique fendue (10a, 110a, 210a) ; et
   le fait d'étendre la fixation mécanique fendue (10a, 110a, 210a) pour fournir des brins (26, 126a, 126b, 1026a) de la fixation mécanique (10b, 110b, 210b) fixés les uns aux autres au moins au niveau de certaines régions de liaison (22) et séparés les uns des autres entre au moins certaines des régions de liaison (22) afin de fournir des ouvertures (24a, 24b, 124a, 124b, 224a, 224b, 1024a, 1024b) ;
   dans lequel la fixation de la fixation mécanique (6, 6a, 8, 8a, 1006, 1008, 3006, 3008) à la couche adhésive (4, 1004, 3004) comprend la fixation des brins (26, 126a, 126b, 1026a) de la fixation mécanique fendue (10b, 110b, 210b) dans une configuration étendue pour maintenir les ouvertures (24a, 24b, 124a, 124b, 224a, 224b, 1024a, 1024b) entre les brins (26, 126a, 126b, 1026a), et dans lequel au moins l'un des brins (26, 126a, 126b, 1026a) constitue la première partie de la fixation mécanique (6, 6a, 1006, 3006) et au moins l'une des ouvertures (24a, 124a, 224a, 1024a) révèle la première partie de la couche adhésive (7, 1007, 3007).

13. Procédé selon la revendication 12, dans lequel au moins l'un de ce qui suit se vérifie : une distance entre au moins certaines des fentes interrompues (20a, 20b, 120, 220, 220b) varie ; une taille des régions de liaison intactes (22) varie entre au moins certaines des fentes interrompues (20a, 20b, 120, 220, 220b), ou la fixation mécanique fendue (10b, 110b, 210b) est étendue non uniformément de sorte qu'au moins certaines des ouvertures (24a, 24b, 124a, 124b, 224a, 224b, 1024a, 1024b) ne sont pas uniformément dimensionnées.

14. Procédé selon la revendication 12 ou 13, dans lequel la première partie de la fixation mécanique (6, 6a, 1006, 3006) est une de plusieurs premières parties de la fixation mécanique (6, 6a, 1006, 3006) fournies par des brins (26, 126a, 126b, 1026a) du support (14, 114, 214) fixés les uns aux autres au niveau de régions de liaison intactes (22) dans le support (14, 114, 214) et séparés les uns des autres entre les régions de liaison (22) de façon à former plusieurs premières ouvertures (24a, 124a, 224a, 1024a) qui révèlent plusieurs premières parties de la couche adhésive (7, 1007) non couvertes par les premières parties de la fixation mécanique (6, 6a, 1006, 3006).

15. Procédé selon l'une quelconque des revendications 12 à 14, dans lequel la deuxième partie (8, 8a, 1008, 3008) de la fixation mécanique est une de plusieurs deuxièmes parties de la fixation mécanique (8, 8a, 1008, 3008) fournies par des brins (26, 126a, 126b, 1026a) du support (14, 114, 214) fixés les uns aux autres au niveau de régions de liaison intactes (22) dans le support (14, 114, 214) et séparés les uns des autres entre les régions de liaison intactes (22) de façon à former des deuxièmes ouvertures (24b, 124b, 224b, 1024b) qui révèlent plusieurs deuxièmes parties de la couche adhésive (7a, 1007a) non couvertes par les deuxièmes parties de la fixation mécanique (8, 8a, 1008, 3008), et dans lequel les deuxièmes ouvertures (24b, 124b, 224b, 1024b) entre les deuxièmes parties de la fixation mécanique (8, 8a, 1008, 3008) sont plus petites que les premières ouvertures (24a, 124a, 224a, 1024a) entre les premières parties de la fixation mécanique (6, 6a, 1006, 3006).

*Fig. 1*

*Fig. 2*

*Fig. 3A*

*Fig. 3B*

*Fig. 4A*

*Fig. 4B*

*Fig. 5A*

*Fig. 5B*

1004    1013    1006    1007    1008    1001

1024b
1007a

1026a

1002    1003    1024a    1026b

**Fig. 6**

2001

2011    2005    2007    2009

2004

2003    2006    2006a    2008    2002

**Fig. 7**

2001

2002    2004    2003    2006a    2008    2009

2011    2006    2007    2007a

**Fig. 8**

Fig. 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 3848594 A, Buell
- US 4001366 A, Brumlik
- US 7407496 B, Peterson
- US 20120204383, Wood
- WO 2005122818 A, Ausen
- WO 199402091 A, Hamilton
- US 20070039142, Petersen
- WO 2011163020 A, Hauschildt
- US 20040147890, Nakahata
- WO 199610481 A, Abuto
- EP 1066008 B1, Eaton
- US 20120330266, Zonneveld
- WO 2012112768 A1
- US 20070173781 A1
- US 4067337 A, Ness
- WO 2013172957 A, Gilbert
- WO 2013172960 A, Gilbert
- US 89128713, Rothwell
- US 20110313389, Wood
- US 5077870 A, Melbye
- US 6132660 A, Kampfer
- US 4775310 A, Fischer
- US 6190594 B, Gorman
- US 7214334 B, Jens
- US 6287665 B, Hammer
- US 7198743 B, Tuma
- US 6627133 B, Tuma
- US 5256231 A, Gorman
- US 5389416 A, Mody